# EUROPEAN PATENT APPLICATION

(11) **EP 3 543 233 A1**
(43) Date of publication of application: **25.09.2019**
(21) Application number: 17853129.9
(22) Date of filing: 21.09.2017
(51) Int. Cl.: C07D 403/14, A61K 31/403, A61P 35/00, A61P 43/00, C12N 15/09, C12Q 1/68

(54) **NOVEL ALKYLATING AGENT**

(30) Priority: 21.09.2016 JP 2016184892
(71) Applicant: Chiba-Prefecture, Chiba-shi, Chiba 260-8667 (JP)
(72) Inventor: NAGASE Hiroki, Tokyo 178-0063 (JP); HATTORI Asuka, Kanonji-shi, Kagawa 768-0061 (JP); WATANABE Takayoshi, Chiba-shi Chiba 260-0801 (JP); TAKATORI Atsushi, Chiba-shi Chiba 260-0804 (JP)
(74) Representative: J A Kemp
(86) International application number: PCT/JP2017/034121
(87) International publication number: WO 2018/056361

(57) **Abstract**

An object of the present invention is to provide a novel alkylating agent that specifically binds to an immune checkpoint gene to induce cell death, and has tumor-specific accumulating properties. The present invention provides a conjugate comprising an alkylating agent bonded to a polyamide that specifically binds to a gene encoding an immune checkpoint molecule, an alkylating agent specific for a gene encoding an immune checkpoint molecule, comprising the conjugate, and a pharmaceutical composition comprising the conjugate.

## Description

### Technical Field

The present invention relates to a novel alkylating agent that alkylates an immune checkpoint gene as a target.

### Background Art

Molecular targeting treatment that targets immune checkpoint molecules has been widely developed (The blockade of immune checkpoints in cancer immunotherapy. Drew M. Pardoll, NATURE REVIEWS CANCER 12; 252-264 2012), and cancer immunotherapy has received attention. It has been difficult so far to develop a molecular targeting therapeutic drug that can maintain long-term remission in solid cancer treatment. However, antibody drugs inhibiting immune checkpoint molecules as targets are clinically applied to various cancers including blood tumor, as a revolutionary treatment method that attains the long-term remission of solid tumor with poor prognosis (Immune checkpoint blockade: a common denominator approach to cancer therapy. Topalian SL, Drake CG, Pardoll DM. Cancer Cell. 2015 Apr 13; 27 (4): 450-61).

Antitumor immunotherapy methods for various cancer types are being established in such a way that programmed cell death 1 (PD-1), programmed cell death ligand 1 (PD-L1), and cytotoxic T-lymphocyte antigen 4 (CTLA-4) as immune checkpoint molecules suppress tumor immunity alone or in combination, thereby restoring tumor-specific T cell immune response. Particularly, laboratory animals or clinical data suggests that treatment with anti-PD-1, anti-PD-Ll, and anti-CTLA-4 antibodies in combination significantly improves treatment outcomes. In addition, as for combination use with radiotherapy or general chemotherapy, it is being revealed that stimulation from dead cells acts on macrophages and the like and improves treatment outcomes (Cell. 2016 Apr 7; 165 (2): 272-5. Combination Cancer Therapies with Immune Checkpoint Blockade: Convergence on Interferon Signaling. Minn AJ, Wherry EJ). In actuality, it has also been reported that combination use of immune checkpoint inhibitors was significantly effective for an actual clinical trial (Postow MA, et al., Nivolumab and ipilimumab versus ipilimumab in untreated melanoma. N Engl J Med. 2015; 372: 2006-17). Furthermore, the findings have been gained that: PD-1 is highly expressed in tumor and also involved in tumor growth; and the suppression of tumor overexpressing PD-1 directly produces a therapeutic effect as the suppression of cancer cell growth (Kleffel et al., Cell 162, 1242-1256 2015).

Meanwhile, the immune checkpoint inhibitors and the anti-PD-1, anti-PD-Ll, and anti-CTLA-4 antibody drugs are systemically administered. Their possibility of causing a serious adverse reaction such as the induction of autoimmune against various organs, tissues, or cells is considered problematic (Nat Rev Rheumatol. 2016 Aug 19. doi: 10.1038/nrrheum.2016.131. Immune checkpoints and rheumatic diseases: what can cancer immunotherapy teach us? van der Vlist M, Kuball J, Radstake TR, Meyaard L). Problems have also been pointed out such as very expensive drugs and the possibility that there is a limitation on tumor that can be treated (Importance of immunopharmacogenomics in cancer treatment: Patient selection and monitoring for immune checkpoint antibodies. Choudhury N, Nakamura Y. Cancer Sci. 2016 Feb; 107 (2): 107-15). In short, many non-responsive cases are also considered problematic (Webster RM. The immune checkpoint inhibitors: where are we now? Nat Rev Drug Discov. 2014; 13 (12): 883-4), and serious adverse reactions such as lethal autoimmune diseases, for example, pulmonary fibrosis and type I diabetes mellitus, are of concern. Hence, there is a demand for a therapeutic drug that specifically inhibits immune checkpoint in tumor and its peripheral microenvironment.

Pyrrole imidazole polyamide (PIP) is an artificial small molecule developed with an antibiotic distamycin as a motif and reportedly binds in a sequence-specific manner to a minor groove of double-stranded DNA. There are mane reports as to research on the silencing of a gene having a target sequence by PIP. Furthermore, PIP is under research as a drug for kidney damage, an anticancer agent, or a therapeutic drug for corneal trauma, hypertrophic scar, bone disease, and the like by animal experiments using mice. Also, the induction of iPS cells by PIP has been researched in recent years at iCeMS, which is the iPS cell project of the Kyoto University, as a part of research on iPS cells. PIP is an organic small molecule expected to produce various research achievements.

Examples of the features of PIP include: (1) PIP can be designed to target an arbitrary gene sequence; (2) its ability to bind to DNA is stronger than that of a transcription factor; (3) PIP is taken up into the nuclei of cells without any vector or drug delivery system (DDS); (4) PIP is stable in cells or organisms without being decomposed by a nucleolytic enzyme and excreted as an undecomposed product by urinary bile; and (5) PIP can be easily modified at its N and C termini and is capable of forming conjugates with various functional small molecules.

PIP recognizes a base pair CG by a Py/Im pair, AT or TA by a Py/Py pair, and GC by an Im/Py pair and is thereby capable of binding to various arbitrary double-stranded DNAs in a sequence-specific manner. PIP bound with the target gene has been studied as a gene switch that inhibits the binding of a transcription factor to DNA and silences the particular gene. An attempt has also been made to develop PIP as a therapeutic drug targeting a particular gene mutation by a conjugate compound with an alkylating agent or the like.

The present inventors have successfully completed: an indole derivative for alkylating a particular nucleotide sequence of DNA by utilizing a PIP structure with high sequence recognition specificity and synthesizing a conjugate of an alkylating functional group and PIP (Patent Literature 2); an invention relating to a target gene-specific histone modification-controlling agent prepared by synthesizing a conjugate of a histone modification-controlling agent and PIP (Patent Literature 1); and the development of a therapeutic drug targeting a driver gene mutation (Patent Literature 3). However, it has not yet been reported that on the basis of the concept of use of PIP, a drug silencing immune checkpoint-related molecules such as PD-1, PD-L1, and CTLA-4 at the same time in a tumor-specific manner (suppressing a mechanism under which cancer evades the immune system) is synthesized and actually produces a tumor suppressive effect.

### Citation List

### Patent Literature

Patent Literature 1: International Publication No. WO2010/001933
Patent Literature 2: International Publication No. WO2005/087762
Patent Literature 3: International Publication No. WO2015/053413

### Summary of Invention

### Technical Problem

As mentioned above, responsible genes of three molecules involved in immune checkpoint, or molecules related thereto are suppressed in combination to promote immune activation through cell death by radiation or chemotherapy. Further, these reactions occur specifically in cancer and a pericancerous microenvironment. There has been a demand for a therapeutic drug that suppresses immune checkpoint molecules in combination in such a manner. Accordingly, an object of the present invention is to provide a novel alkylating agent that alkylates PD-1, PD-L1, and CTLA-4 genes as targets to silence these gens while directly inducing cancer cell death, more effectively suppresses the peritumoral expression of the immune checkpoint genes by the accumulation of the agent in tumor, and minimizes action on other organs.

### Solution to Problem

The present inventors have conducted diligent studies to attain the object and consequently, successfully synthesized a novel compound that specifically recognizes, binds to, and alkylates genes of immune checkpoint molecules PD-1, PD-L1, and CTLA-4, by designing pyrrole imidazole polyamide (PIP) CCC07-01 that specifically binds to the 3 genes of PD-1, PD-L1, and CTLA-4, and bonding an alkyl reaction site to the designed PIP to synthesize the compound. It has been further confirmed that the novel compound efficiently silences the PD-1, PD-L1, and CTLA-4 genes and induces the cell death of tumor cells. From the material properties of the compound, findings suggesting uptake into tumor cells and accumulating properties in tumor transplanted in mice have been gained. Therefore, the present invention has been completed.

Specifically, the present invention is as follows:
(1) A conjugate of a DNA-binding compound and an alkylating agent, wherein the DNA-binding compound specifically binds to a gene region encoding an immune checkpoint molecule or a related molecule thereof.
(2) The conjugate according to (1), wherein the conjugate accumulates in tumor and a peritumoral microenvironment and specifically suppresses the immune checkpoint molecule or the related molecule thereof in the tumor.
(3) The conjugate according to (1) or (2), wherein the immune checkpoint molecule is selected from the group consisting of PD-1, PD-L1, CTLA-4, CD80, CD86, PD-L2, and CD28, and the molecule related to the immune checkpoint molecule is at least one or more members selected from the group consisting of LAG3, TIGIT, IFNγ, IFN-I, IDO, KIR2DL-1, KIR2DL-2, KIR2DL-3, KIR2DS-1, KIR2DA-2, B7-H3 (CD276), TIM-3, VISTA, B7RP1, ICOS, B7-H4, HVEM, BTLA, CD137, CD70, OX40, CD40, CD137L, CD70L, OX40L, CD40L, GAL9, A2eR, TGFβ, IL1, IL6, IL10, IL12 and IL18, and immune-related molecules registered in gene databases.
(4) The conjugate according to any one of (1) to (3), wherein the DNA-binding compound is selected from the group consisting of a bridged nucleic acid, a locked nucleic acid (LNA), PNA, pyrrole imidazole polyamide (PIP), a modified form of pyrrole imidazole polyamide (PIP), a DNA-binding protein, and a DNA-binding protein complex.
(5) The conjugate according to any one of (1) to (4), wherein the alkylating agent is a compound having a functional group having the ability to alkylate a particular nucleotide sequence of DNA.
(6) The conjugate according to (5), wherein the alkylating agent is secoCBI.
(7) An alkylating agent specific for a gene encoding an immune checkpoint molecule or a related molecule thereof, the alkylating agent comprising a conjugate according to any one of (1) to (6).
(8) The conjugate according to (1), wherein the conjugate is represented by the following formula: 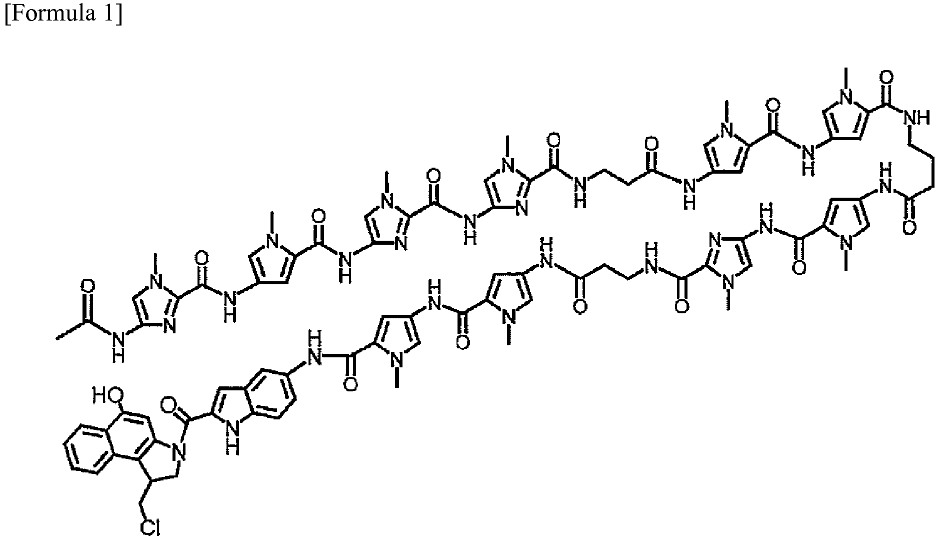
(9) The conjugate according to (1), wherein the conjugate is represented by the following formula: 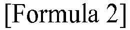 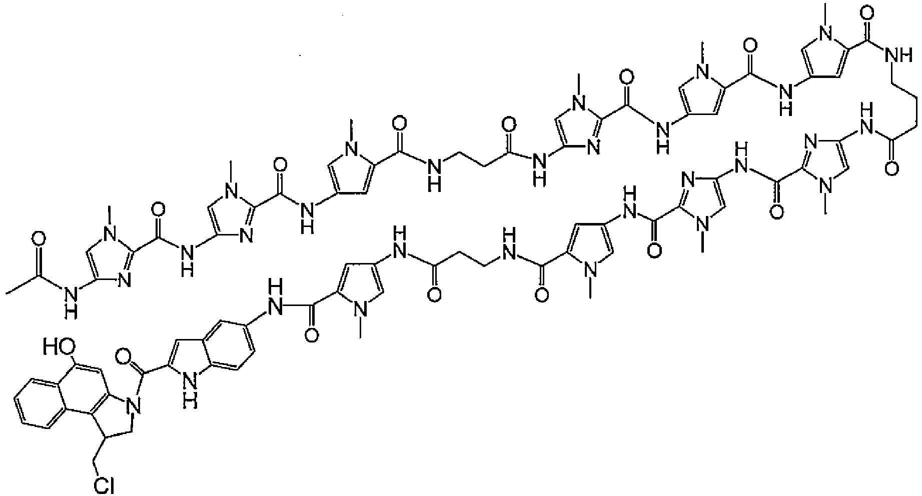
(10) The conjugate according to (1), wherein the conjugate is represented by the following formula: 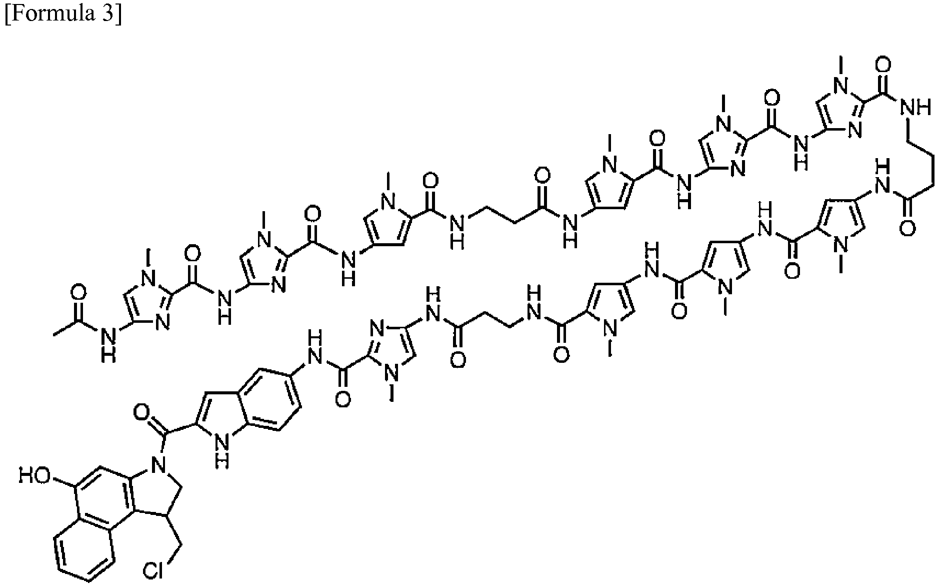
(11) An alkylating agent that binds to a gene region encoding an immune checkpoint molecule, the alkylating agent comprising a conjugate according to any of (8) to (10).
(12) An alkylating agent for a PD-1 molecule gene, the alkylating agent comprising a conjugate according to any of (8) to (10).
(13) An alkylating agent for a PD-L1 molecule gene, the alkylating agent comprising a conjugate according to any of (8) to (10).
(14) An alkylating agent for a CTLA-4 molecule gene, the alkylating agent comprising a conjugate according to any of (8) to (10).
(15) An alkylating agent capable of binding to gene regions of two or more of PD-1, PD-L1, and CTLA-4 molecule genes, the alkylating agent comprising a conjugate according to any of (8) to (10).
(16) A pharmaceutical composition comprising a conjugate according to any one of (1) to (6) and (8) to (10).
(17) The pharmaceutical composition according to (16), wherein the pharmaceutical composition is an anticancer agent.
(18) A kit comprising a conjugate according to any one of (1) to (6) and (8) to (10).
(19) A research reagent kit comprising a conjugate according to any one of (1) to (6) and (8) to (10).
(20) A kit for treatment comprising a conjugate according to any one of (1) to (6) and (8) to (10).
(21) A method for producing a conjugate that specifically alkylates a gene encoding an immune checkpoint molecule or a related molecule thereof, comprising the steps of:
   (1) designing a DNA-binding compound so as to specifically bind to the gene encoding an immune checkpoint molecule or a related molecule thereof; and
   (2) bonding the designed DNA-binding compound to an alkylating agent.
(22) The method for producing a conjugate according to (21), wherein the gene region is an exon or an intron, and a promoter or a 3 prime transcriptional region.
(23) The method for producing a conjugate according to (21) or (22), wherein the immune checkpoint molecule is at least one member selected from PD-1, PD-L1, CTLA-4, CD80, CD86, PD-L2, and CD28, and genes involved in immune response, or at least one combination selected from the group consisting of combinations of two or more of the molecules.
(24) The method for producing a conjugate according to any one of (21) to (23), wherein the DNA-binding compound is selected from the group consisting of a bridged nucleic acid, a locked nucleic acid (LNA), PNA, pyrrole imidazole polyamide (PIP), a modified form of pyrrole imidazole polyamide (PIP), a DNA-binding protein, and a DNA-binding protein complex.
(25) The method for producing a conjugate according to any one of (21) to (24), wherein the alkylating agent is a compound having a functional group having the ability to alkylate a particular nucleotide sequence of DNA.
(26) The method for producing a conjugate according to (25), wherein the alkylating agent is secoCBI.

The present specification encompasses the contents described in the specification and/or drawings of Japanese Patent Application No. 2016-184892 on which the priority of the present application is based.

### Advantageous Effects of Invention

The present invention provides a conjugate for specifically alkylating a gene region of an immune checkpoint molecule in tumor cells and a peripheral microenvironment. According to a preferred aspect of the present invention, the conjugate of the present invention can be used as a pharmaceutical composition or an anticancer agent. According to an alternative preferred aspect of the present invention, the conjugate of the present invention is a compound that specifically alkylates an immune checkpoint molecule gene. According to an alternative preferred aspect of the present invention, the conjugate of the present invention is a compound that specifically alkylates and silences an immune-related gene in tumor.

The present invention has revealed that a methodology of adding a polyamide that specifically binds to a gene of an immune checkpoint molecule is a very effective approach for the creation of a novel anticancer agent by which immunocytes recognize tumor-specific various proteins. Specifically, an anti-immune checkpoint molecule silencing therapeutic drug that has strong binding activity against DNA and effectively attains gene silencing even in a small amount can be developed by adding an alkylating agent CBI to a nucleotide sequence-specific binding organic small molecule PIP. This effect is exerted probably because DNA damage ascribable to alkylation induces a cell death signal and releases tumor-specific antigenic proteins in tumor cells so that the activation of tumor immunity involving cancer cells usually inducing no tumor immunity can be induced.

In short, the present invention provides a technique of delivery to the genomic sequence of an immune checkpoint molecule. The application of this technique also enables design and synthesis of one therapeutic drug after another that can specifically recognize the consensus sequences of immune checkpoint molecules and mutated sequences of not only immune checkpoint molecule genes but cancer-specific various cancer-related genes. Furthermore, a concept similar to the antibody-drug conjugate (ADC) technology can also be applied thereto, and the synthesis is also easy. Therefore, the technique of the present invention can be applied to a wide range of purposes.

The conjugate of the present invention has the excellent advantages that: the conjugate specifically silences an immune checkpoint molecule gene at a concentration equal to or lower than a concentration inducing cell death, for a cell line expressing the immune checkpoint molecule gene; the conjugate further exhibits anticancer activity (causes the suppression of cell growth from nanomole); the conjugate is automatically synthesizable; the conjugate has high accumulating properties in tumor; and the conjugate is localized in the nucleus. Hence, the conjugate of the present invention not only provides a tumor-specific strong immune checkpoint molecule inhibitor that accumulates in tumor and a peritumoral microenvironment while avoiding clinically problematic autoimmune diseases against organs, tissues, or cells, but enables development of a novel revolutionary therapeutic drug as a therapeutic drug targeting immune checkpoint molecules in combination. The conjugate of the present invention is expected to pursue research and development (physical property test, safety test, pharmacokinetics, large-scale synthesis in GMP) toward the initiation of clinical trials in the future. The application of the method of the present invention also enables further development of a revolutionary therapeutic drug that exerts a similar or additive and/or synergistic effect.

### Brief Description of Drawings

Figure 1 is a diagram showing the structure of PD-1 gene and its binding position to a compound.
Figure 2 is a diagram showing the structure of PD-L1 gene and its binding position to a compound.
Figure 3 is a diagram showing the structure of CTLA-4 gene and its binding position to a compound.
Figure 4-1 is a diagram showing HPLC (Figure 4-1A) and LC-MS (Figure 4-1B) chromatograms with regard to the synthesis of a PIP-drug conjugate that binds to and alkylates three gene regions of immune checkpoint molecules PD-1, PD-L1, and CTLA-4.
Figure 4-2 is a diagram showing results of WST assay in which IC50 was calculated.
Figure 5 is a diagram showing results of an experiment to confirm the silencing of the PD-1 gene by the administration of the PIP-drug conjugate to a cell line expressing the immune checkpoint molecule PD-1. Figure 5A shows an agarose electrophoretic profile, and Figure 5B shows results of quantification by real-time PCR.
Figure 6 is a diagram showing results of an experiment to confirm the silencing of the PD-L1 gene by the administration of the PIP-drug conjugate to a cell line expressing the immune checkpoint molecule PD-L1. Figure 6A shows an electrophoretic profile, and Figure 6B shows results of quantification by real-time PCR.
Figure 7 is a diagram showing results of an experiment to confirm the silencing of the CTLA-4 gene by the administration of the PIP-drug conjugate to a cell line expressing the immune checkpoint molecule CTLA-4. Figure 7A shows an agarose electrophoretic profile,
Figure 7B shows results of quantifying expression in MIA PaCa-2 cells, and Figure 7C shows results of quantifying expression in A2058 cells.
Figure 8 is a diagram showing results of performing the cell growth measurement of human large intestine cancer cell line SW480 and RKO cells after treatment with the PIP-drug conjugate or a CBI alkylating agent by WST assay, and measuring IC50 (50% inhibitory concentration). Figure 8A shows the results about CCC07-01, and Figure 8B shows the results about CBI.
Figure 9 is a diagram showing results of observing uptake into cultured tumor cells 2 hours after administration of FITC-labeled PIP having a distinctive log-P value.
Figure 10 is a diagram showing results of observing tumor tissue accumulation and storage and excretion from a normal organ 72 hours after intravenous administration of FITC-labeled PIP having a distinctive log-P value to mice.
Figure 11 is a diagram showing the structures of 6 types of PI polyamides (Im00 to Im05).
Figure 12 is a diagram showing the retention times of 4 types of compounds (ethyl acetate, benzonitrile, acetophenone, and indole) and a calibration curve determined from log-P values.
Figure 13 is a diagram showing the retention times of 2 types of compounds (indole and curcumin) and a calibration curve determined from log-P values.
Figure 14 is a diagram showing change in tumor volume when CCC07-01 was administered to human tumor-transplanted mice.
Figure 15 is a diagram showing the ratio of cytotoxic T cells to Treg in the spleen when CCC07-01 was administered to human tumor-transplanted mice.
Figure 16 is a diagram showing the structural formula of CCC07-03.
Figure 17 is a diagram showing HPLC analysis data on CCC07-03.
Figure 18 is a diagram showing LC-MC analysis data on CCC07-03.
Figure 19 is a diagram showing the structural formula of CCC07-04.
Figure 20 is a diagram showing HPLC analysis data on CCC07-04.
Figure 21 is a diagram showing LC-MC analysis data on CCC07-04.
Figure 22 is a diagram showing results of quantifying expression after CCC07-03 administration in RKO cells expressing PD-L1. Figure 22A shows the results obtained 6 hours later, Figure 22B shows the results obtained 24 hours later, and Figure 22C shows the results obtained 48 hours later.
Figure 23 is a diagram showing results of quantifying expression after CCC07-03 administration in A2058 cells expressing CTLA-4. Figure 23A shows the results obtained 6 hours later, and Figure 23B shows the results obtained 24 hours later.
Figure 24 is a diagram showing results of quantifying expression after CCC07-04 administration in A2058 cells expressing CTLA-4.
Figure 25 is a diagram showing results of quantifying expression after CCC07-04 administration in B16F1 mouse cells expressing Pd-l1.
Figure 26 is a diagram showing the accumulating properties of PI polyamide compounds in the tumor of cancer cell-transplanted nude mice.
Figure 27 is a diagram showing fluorescent staining images showing the accumulating properties of PI polyamide compounds in the tumor of cancer cell-transplanted nude mice.

### Description of Embodiments

Hereinafter, the present invention will be described in detail. All publications cited herein, for example, prior art documents, publication bulletins, patent bulletins, and other patent literatures, are incorporated herein by reference.

The present invention provides a conjugate of a DNA-binding compound and an alkylating agent, wherein the DNA-binding compound specifically binds to a gene region encoding an immune checkpoint molecule or a related molecule thereof.

Examples of the DNA-binding compound that specifically binds to the gene region of an immune checkpoint molecule or a related molecule thereof include compounds having a PIP (pyrrole imidazole polyamide) structure.

It has already been known that a gene sequence-specific alkylating agent can be synthesized using a PIP structure with high sequence recognition specificity. However, it has been uncertain whether PIP can be applied as a drug to the inhibition of an immune checkpoint molecule on the basis of this concept. It has also been reported that a gene having a target sequence can be silenced using a compound of an alkylating agent conjugated with PIP. However, the fact has not yet been known that a drug targeting immune checkpoint molecules in combination can be actually synthesized and can produce a tumor suppressive effect. The present inventors have hypothesized that: an alkylating agent induces DNA damage, thereby inducing cell death, triggering a mechanism to suppress PD-1 promoting tumor growth, and releasing an intracellular tumor antigen from cancer cells; and the compound accumulates in tumor and sustainably suppresses immune checkpoint molecules in combination in the tumor and a peripheral microenvironment, thereby effectively inducing the inhibition of immune checkpoint to the tumor microenvironment so that the antitumor effect of cancer immunotherapy is maximized while an immune checkpoint inhibitory effect in organs, tissues, or cells throughout the body is reduced, whereby therapeutically problematic autoimmune disease such as type I diabetes mellitus or hypothyroidism can be prevented.

On the basis of this hypothesis, a PIP-drug conjugate has been synthesized, and its influence on gene expression has been observed in cancer cell lines highly expressing immune checkpoint molecules PD-1, PD-L1, and CTLA-4, respectively. As a result, the conjugate was able to be also confirmed to specifically transcriptionally silence, at a very low concentration, the gene of each immune checkpoint molecule. Furthermore, it has been predicted that the conjugate can induce cell death and is effectively taken up into cultured cells to effectively accumulate in the tumor of human large intestine cancer transplanted in mice. Thus, the compound CCC07-01 of the present invention, which is a PIP-drug conjugate, has been found to suppress immune checkpoint molecules in combination and to serve as an anticancer agent that can produce anticancer properties.

These results have demonstrated that: a sequence-specific gene-recognizing polyamide compound can be synthesized; and an anticancer agent targeting immune checkpoint molecules in combination can be synthesized by use of a method for synthesizing a conjugate with a drug that can induce the alkylation of DNA.

Organisms have a mechanism for recognizing pathogens that have invaded their bodies or cells or organic bodies that have invaded or developed in their bodies, such as cancer cells, as non-self, and eliminating these substances. This is called immunity. Cancer cells are recognized as non-self, killed and eliminated by cytotoxic T cells (CTL) or the like which attack the cancer cells. Receptor proteins and ligand proteins for decreasing the activity of this CTL reside on cell surface and suppress immune response. This mechanism is "immune checkpoint". The recognition of cancer cells as non-self requires presenting a new antigen that is recognized by immunocytes. Cancer cells present a so-called cancer antigen by the accumulation of a gene mutation, the induction of specific gene expression, etc., and are thereby recognized as non-self.

Research on the inhibition of mouse cytotoxic T lymphocyte antigen 4 (CTLA-4) has demonstrated that provided that this immune checkpoint is inhibited, immunosuppression attributed to cancer cells is canceled so that CTL can be activated to kill the cancer cells (Leach, D.R., Krummel, M.F., and Allison, J.P. (1996). Enhancement of antitumor immunity by CTLA-4 blockade. Science 271, 1734-1736). The inhibitor has also been proved effective for clinical trials for malignant melanoma in humans and has been approved as a drug (Hodi, F.S., O'Day, S.J., McDermott, D.F., Weber, R.W., Sosman, J.A., Haanen, J.B., Gonzalez, R., Robert, C., Schadendorf, D., Hassel, J.C., et al., (2010). Improved survival with ipilimumab in patients with metastatic melanoma. N. Engl. J. Med. 363, 711-723). Inhibitors such as antibody drugs against immune checkpoint molecules have been developed, and their clinical application has already been started. Furthermore, for example, compounds inhibiting immune checkpoint pathways such as LAG3, TIGIT, IFNγ, IFN-I, IDO, KIR2DL-1, KIR2DL-2, KIR2DL-3, KIR2DS-1, KIR2DA-2, B7-H3 (CD276), TIM-3, and VISTA as targets have been developed and tested both clinically and non-clinically (Immune checkpoint blockade: a common denominator approach to cancer therapy. Topalian SL, Drake CG, Pardoll DM. Cancer Cell. 2015 Apr 13; 27 (4): 450-61). In addition, immune checkpoint-related molecules such as B7RP1, ICOS, B7-H4, HVEM, BTLA, CD137, CD70, OX40, CD40, CD137L, CD70L, OX40L, CD40L, GAL9, A2eR, TGFβ, IL1, IL6, IL10, IL12, and IL18 are also under research (The blockade of immune checkpoints in cancer immunotherapy. Drew M. Pardoll Nature Reviews Cancer 12; 252-264 2012).

The immune checkpoint molecule refers to a receptor protein or a ligand protein that regulates CTL activity in cancer cells and in immunocytes present in the peripheral microenvironment of the cancer cells. The immune checkpoint molecule includes PD-1, PD-L1, CTLA-4, CD80, CD86, PD-L2, and CD28. Examples of the molecule related to the immune checkpoint molecule include LAG3, TIGIT, IFNγ, IFN-I, IDO, KIR2DL-1, KIR2DL-2, KIR2DL-3, KIR2DS-1, KIR2DA-2, B7-H3 (CD276), TIM-3, VISTA, B7RP1, ICOS, B7-H4, HVEM, BTLA, CD137, CD70, OX40, CD40, CD137L, CD70L, OX40L, CD40L, GAL9, A2eR, TGFβ, IL1, IL6, IL10, IL12, IL18, and immune-related molecules registered in gene databases. These molecules are under research on therapeutic drugs for cancer as targets for canceling immunosuppression attributed to cancer cells.

In a phase III clinical trial on malignant melanoma by combination treatment targeting PD-1 and CTLA-4, a nivolumab (anti-PD-1 antibody) + ipilimumab (anti-CTLA-4 antibody) combination use group had an objective response rate (ORR) of 60% whereas an ipilimumab (anti-CTLA-4 antibody) alone group had an objective response rate (ORR) of 11%, showing significant improvement in the combination use group. This combination treatment has been approved. For kidney cancer or non-small cell lung cancer, a clinical trial has also been conducted by this treatment. Furthermore, a clinical trial has been attempted on combination use for the inhibition of CTLA-4 and PD-L1, or combination treatment for the inhibition of PD-1 and LAG-3 or PD-1 and PD-L1. An effect brought about by the inhibition of immune checkpoint molecules in combination, particularly, combination use of a PD-1/PD-L1 pathway and a CTLA-4 pathway is currently drawing most attention, and various clinical trials are underway (Combined Immune Checkpoint Blockade: Charles G. Drak Seminars in Oncology, 42 (4) 656-662 2015; and Combination Cancer Therapies with Immune Checkpoint Blockade: Convergence on Interferon Signaling. Minn AJ, Wherry EJ Cell. 165 (2): 272-5 2016).

Some cases are responsive to immune checkpoint inhibitors, and others are not. Therefore, the possibility of developing an optimum cancer treatment method utilizing the inhibition of immune checkpoint has been proposed from the analysis of responsive cases and furthermore, the analysis of combination treatment methods that theoretically produce an additive or synergistic effect in animal experiments or clinical trials. Anticancer treatment by the inhibition of immune checkpoint requires the presence of a cancer antigen molecule, the enhancement of tumor antigen-specific CD8-positive T cells and IFN type I- and IFN-γ-producing cells, and a mechanism to negatively regulate the inhibitory signals of CD4-positive regulatory T cells. The induction of the cell death of tumor cells or vaccination with a tumor antigen enhances the presentation of a tumor-specific antigen. For example, radiation or treatment with an anticancer agent induces cell death from DNA damage and activates tumor immunity. Dead cells release MHC class I substances to stimulate CD8-positive T cells and to secrete IFN type I from tumor-related macrophages, for example, thereby activating antitumor immunity. This is considered to enhance the antitumor effects of immune checkpoint inhibitors (Combination Cancer Therapies with Immune Checkpoint Blockade: Convergence on Interferon Signaling. Minn AJ, Wherry EJ Cell. 165 (2): 272-5 2016; and Radiation and checkpoint blockade immunotherapy: radiosensitisation and potential mechanisms of synergy. Andrew B Sharabi, Michael Lim, Theodore L DeWeese, Charles G Drake, Lancet Oncology 2015 16: e498-509).

The inhibition of immune checkpoint eliminates cancer by immunotherapy which utilizes the original immune system of humans, and has been expected as a treatment method with fewer adverse reactions. In actuality, from results of clinical trials, response rates for advanced cancer are high and greatly exceed the frequency of serious adverse reactions. Therefore, immune checkpoint inhibitors, albeit having adverse reactions, have been approved as drugs. Adverse events (pulmonary toxicity, pancreatic toxicity, gastrointestinal toxicity, hepatic toxicity, endocrine toxicity, dermal toxicity, renal toxicity, neurotoxicity, etc.) probably caused by autoimmune occur as adverse reactions. Particularly, type I diabetes mellitus ascribable to pancreatic endocrine toxicity develops acutely and becomes lethal, or adverse reactions, such as interstitial pneumonia or hypothyroidism, which require attention are found with given frequency. Antibody drugs are systemically administered and act on cells of the immune system having immune checkpoint molecules throughout the body. Therefore, the immune checkpoint is also suppressed in the organs described above so that immune response to own tissues is activated to attack normal organ cells, probably resulting in toxicity in the organs or endocrine cells.

The specific delivery of a drug to tumor tissues has already been attempted using drugs such as Photofrin or Laserphyrin for use in, for example, antibody-drug conjugates (ADCs) or photodynamic therapy (PDT), and has already been widely used in clinical practice as a treatment method which involves delivering the drug into cells by accumulation in tumor, or activating the drug after gradual accumulation of the drug in tumor cells and sufficient excretion from normal tissues, to induce the cell death of the tumor cells. Provided that the drug can be allowed to accumulate in tumor, it is considered that tumor immunity can be activated while the induction of autoimmune against other normal organs or tissues is minimized. Thus, it is considered that the suppression of immune checkpoint can alleviate the adverse reaction of organ toxicity ascribable to activated immune response to own tissues.

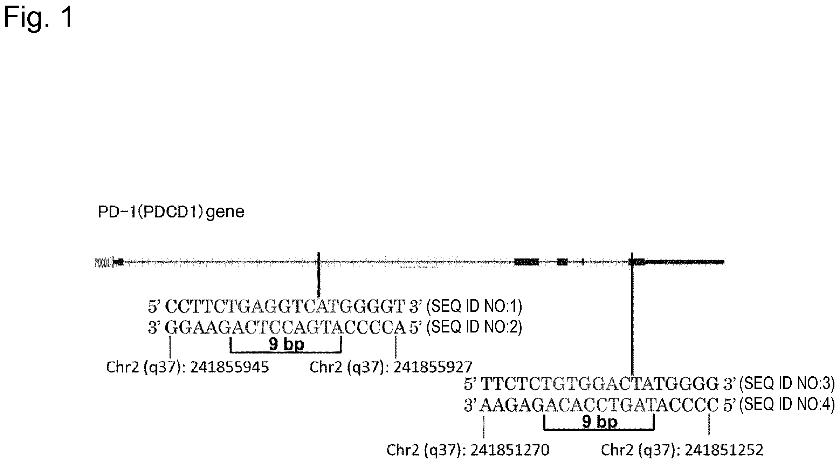

The PD-1 gene encodes a PD-1 (programmed cell death 1) molecule and was isolated and identified in 1992 as a gene whose expression is enhanced at the time of induction of the cell death of T cells. Autoimmune develops in PD-1-deficient mice, indicating the possibility that cancer can be attacked by blocking PD-1 signals. It was further found that: cancer overexpresses PD-L1 and causes immunosuppression; and the PD-1 pathway is also important for the suppression of tumor immunity because antitumor activity was obtained by administering PD-1 and PD-L1 antibodies to cancer-bearing mice. Then, a PD-1 antibody was the first of these agents to be approved in Japan, as a therapeutic drug for malignant melanoma, in July 2014. The response rate of treatment with the PD-1 antibody alone was 20 to 30%, whereas its combination treatment with a CTLA-4 antibody drastically improved the response rate to 50 to 60%, depending on cancer type. It has also been reported that: PD-1 is also overexpressed in some cancers and thereby involved in cancer growth; and tumor growth can be suppressed by the suppression of PD-1. Thus, PD-1 contributes to cancer growth through a non-immune pathway (Kleffel et al., Cell 162, 1242-1256 2015). However, little is known about the reason why the other patients are nonresponsive to treatment with the PD-1 antibody, and about combination treatment that improves the response rate. Figure 1 shows the genomic structure of the PD-1 gene.

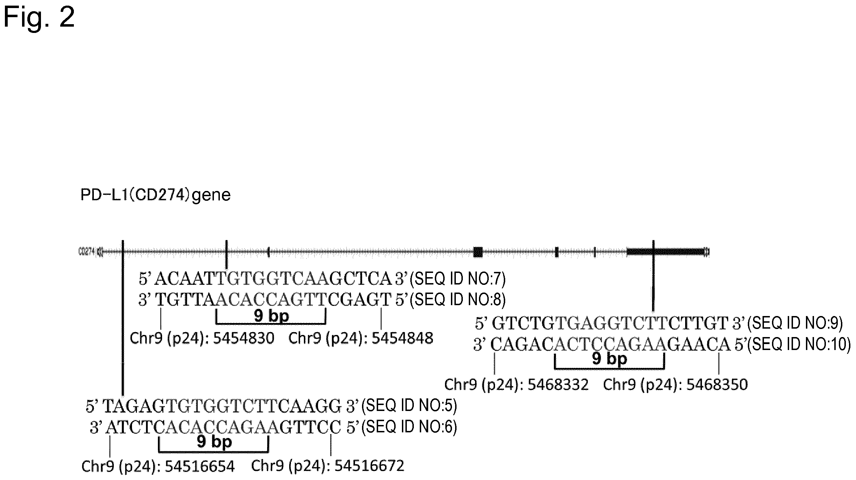

The PD-L1 gene encodes a principal ligand of PD-1 and is overexpressed in some cancers. PD-L1 negatively regulates tumor immunity and promotes the evasion of tumor from the immunity. Although PD-L2 is also known as a ligand of PD-1, PD-L2 is known to also positively regulate tumor immunity. An anti-PD-Ll antibody had been widely tested as an immune checkpoint inhibitor and was approved for locally progressive or metastatic urothelial cancer in 2016. However, infection and autoimmune disease-like symptoms (pneumonia, hepatitis, enteritis, thyroiditis, adrenal insufficiency, pancreatitis, eruption or dermatitis, etc.) have been found as adverse reactions of grade 3 or higher in 50% of cases. Still, adverse reactions in normal organs are problematic. The therapeutic antibody drug targeting PD-L1 exhibits no significant correlation with the expression of PD-L1 and is found effective even for cases without the expression of PD-L1. Figure 2 shows the genomic structure of the PD-L1 gene.

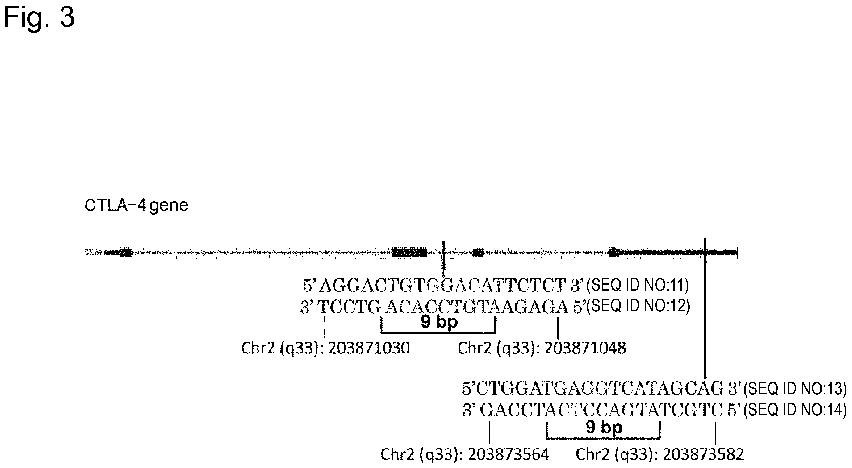

The CTLA-4 gene encodes a cell surface molecule commonly expressed by activated T cells or regulatory T cells (Treg). CTLA-4 is a negative costimulatory receptor and is similar to a positive costimulatory receptor CD28. CTLA-4 and CD28 share ligands (CD80 and CD86). CTLA-4 is a molecule suppressing autoimmune functions and also negatively regulates tumor immunity by the growth and activation of tumor antigen-specific T cells and decrease in regulatory T cells Treg. An anti-CTLA-4 antibody was the first of these agents to be approved, as an immune checkpoint inhibitor, for malignant melanoma by US FDA in 2011. Then, it has been reported that its combination use with an anti-PD-1 antibody drastically improves a response rate. However, a problem of this antibody drug is that immune-related adverse reactions occur mainly in the skin (dermatitis and pruritus), the gastrointestinal tract (diarrhea and colitis), the liver (hepatic dysfunction and hepatitis), the endocrine gland (adenohypophysitis, aberrations of the adrenal gland, aberrations of the thyroid gland, etc.), the nervous system (peripheral neuropathy, etc.) and other organs (interstitial pneumonia, uveitis, episcleritis, nephritis, etc.) during an administration period or even after discontinuation of administration, as in the inhibition of the PD-1 pathway. Figure 3 shows the genomic structure of the CTLA-4 gene.

Under these circumstances, an effective drug that suppresses immune checkpoint molecules in combination, furthermore, can specifically suppress the immune checkpoint molecules around tumor, and can induce tumor cell death has not yet been developed.

The immune checkpoint gene-recognizing polyamide serving as a component of the conjugate of the present invention is a polyamide designed so as to recognize the sequence of the immune checkpoint gene. The phrase "recognizing an immune checkpoint gene" means that the immune checkpoint gene-recognizing polyamide binds (e.g., by a hydrogen bond or cross-linking), for example, to the nucleotide sequence of the immune checkpoint gene and/or the neighborhood thereof. Examples of the immune checkpoint gene-recognizing compound can include DNA-binding compounds such as pyrrole imidazole polyamide (PIP) (PIP compounds including a cyclic structure, a hairpin structure, and a tandem form in which hairpin structures are connected), peptide nucleic acids (PNAs), bridged nucleic acids, locked nucleic acids (LNAs), DNA-binding proteins (e.g., zinc finger) and chimeric proteins thereof, guide RNA-protein complexes. Furthermore, a modified form of PIP modified so as to maintain or improve the ability to bind to DNA is also included in the present invention. Examples of the modified form of PIP include a modified form containing an amine added to position α or β of the γ-aminobutyric acid of PIP, a modified form having a substituted side chain of N-α-N-γ-aminobutyric acid or N-β-N-γ-aminobutyric acid, modified forms derived from the modified forms described above by modification with a molecule such as fluorescein isothiocyanate (FITC) or biotin, a modified form of PIP modified at its N terminus with a molecule such as FITC or biotin, and a modified form of PIP modified at its C terminus with a molecule such as isophthalic acid.

The pyrrole imidazole polyamide (PIP) (also referred to as PI polyamide) is a polyamide containing a N-methylpyrrole unit (Py), a N-methylimidazole unit (Im), and a γ-aminobutyric acid moiety, and Py, Im, and the γ-aminobutyric acid moiety are linked to each other via an amide bond (-C(=O)-NH-) (Trauger et al., Nature, 382, 559-61 (1996); White et al., Chem Biol., 4, 569-78 (1997); and Dervan, Bioorg. Med. Chem., 9, 2215-35 (2001)). PIP is wholly folded into a U-shaped conformation (hairpin form) by the γ-aminobutyric acid moiety serving as a linker (γ-linker). In the U-shaped conformation, two chains containing Py and Im are arranged in parallel, flanking the linker. When a pair of Py and Im between these two chains is a particular combination (Py/Im pair, Im/Py pair, Py/Py pair, or Im/Im pair), this pair can bind to a particular base pair in DNA with high affinity. For example, the Py/Im pair can bind to a C-G base pair, and the Im/Py pair can bind to a G-C base pair. Also, the Py/Py pair can bind to both an A-T base pair and a T-A base pair (White et al., Chem. Biol., 4, 569-78 (1997); and Dervan, Bioorg. Med. Chem., 9, 2215-35 (2001)). PIP may additionally contain 3-hydroxypyrrole (Hp) or β alanine. As for Hp, a Hp/Py pair can bind to a T-A base pair (White et al., Nature, 391, 468-71 (1998)). The γ-linker may have a side chain having an amino group, such as N-α-N-γ-aminobutyric acid and N-β-N-γ-aminobutyric acid, which may be modified with a molecule such as FITC or biotin. Also, PIP may be modified at its N terminus with not only an acetyl group but a molecule such as FITC or biotin. β alanine/β alanine can bind to a T-A base pair or an A-T base pair. Accordingly, PIP recognizing the regulatory region of the target gene can be designed by changing, for example, the pairing combination of Py and Im according to the DNA sequence of the target. A design method and a production method for PIP are known in the art (e.g., JP Patent No. 3045706, JP Patent Publication (Kokai) No. 2001-136974 A (2001) and International Publication No. WO03/000683).

PIP can recognize a gene sequence. Thus, a polyamide has been designed using a pair of imidazole and pyrrole recognizing guanine and cytosine and a pair of pyrrole and β alanine recognizing adenine and thymine, in order to recognize, as shown in Figure 1, for example, a TGAGGTCAT sequence positioned from PD-1 intron 1 chromosome 2 (q37): 241855932 to 241855940 and a TGTGGACTA sequence positioned from PD-1 exon 5 chromosome 2 (q37): 241851257 to 241851265 in Human Dec. 2013 (GRCh38/hg38) Assembly. Also, the polyamide has been synthesized by locating an indole group between a pyrrole group and an alkylating agent secoCBI such that an alkylation site is located close to N3 of adenine on the basis of structure analysis, so as to obtain a good angle. Furthermore, the polyamide has been designed by making a consideration to ensure the alkylation of the transcription template DNA of PD-1. Thus, as for nucleotide substitution, it may be expected that an antitumor effect can also be obtained by utilizing the ability of PIP to recognize bases or the ability of secoCBI to recognize adenine, and thereby synthesizing a compound that can recognize even the sequences of various immune checkpoint-related genes.

The bridged nucleic acid or the locked nucleic acid (LNA) can be designed so as to recognize the regulatory region of the target gene, and synthesized as 2',4'-BNA in which an oxygen atom at position 2' and a carbon atom at position 4' of RNA are bridged via a methylene chain, or as 2',4'-ENA (ethylene-bridged nucleic acid) in which an oxygen atom at position 2' and a carbon atom at position 4' of RNA are bridged via an ethylene chain. LNA is also available from Proligo LLC.

Specifically, the DNA-binding compound in the conjugate of the present invention is selected from the group consisting of a bridged nucleic acid, a locked nucleic acid (LNA), PNA, pyrrole imidazole polyamide (PIP), a modified form of pyrrole imidazole polyamide (PIP), a DNA-binding protein, and a DNA-binding protein complex.

PIP having higher lipid solubility is more preferred because of having higher tumor accumulating properties. PIP having higher log-P (octanol/water partition coefficient) has higher lipid solubility.

The alkylating agent includes a compound having a functional group alkylating DNA. The alkylating agent can be any alkylating agent without particular limitations as long as the alkylating agent comprises a compound having both of the ability to alkylate a particular nucleotide sequence present in DNA and the ability to recognize the sequence. For example, a compound containing a functional group described in International Publication No. WO2010/001933 can be used. The alkylating agent introduces an alkyl group to the N-7 position of guanine or the N-3 position of adenine in DNA to cause adduct formation or cross-linking reaction between guanines, between adenines, etc. The double-stranded DNA that has undergone cross-linking reaction or adduct formation is difficult to repair by a repair mechanism. As a result, the DNA can be no longer transcribed or replicated to induce cell death ascribable to cell growth arrest or apoptosis. The DNA-binding protein includes a binding protein such as zinc finger, a chimeric protein such as TALEN (TALE nuclease), and a complex protein such as CRISPR utilizing a guide nucleic acid.

Examples of the alkylating agent include secoCBI (1-chloromethyl-5-hydroxy-1,2-dihydro-3H-benzo[e]indole). The secoCBI (1-chloromethyl-5-hydroxy-1,2-dihydro-3H-benzo[e]indole) is a compound represented by the formula given below. The secoCBI is a compound known in the art to alkylate DNA, and specifically alkylates adenine on the minor groove side of DNA bases. The secoCBI can be synthesized by a method described in a literature known in the art ((a) Boger, D.L.; Yun, W.Y.; Teegarden, B.R. J. Org. Chem. 1992, 57, 2873; (b) Boger, D.L.; McKie, J.A. J. Org. Chem. 1995, 60, 1271; and (c) Boger, D.L.; Ishizaki, T.; Kitos, P.A.; Suntornwat, O. J. Org. Chem. 1990, 55, 5823).

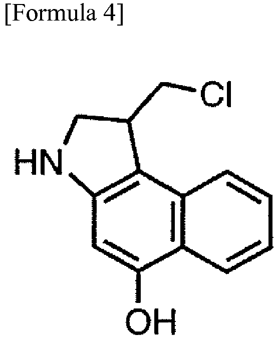

The conjugate of the present invention can be synthesized, for example, by bonding the polyamide to the alkylating agent. The synthesis method can be performed by a method known in the art (J. Am. Chem. SOC. 1995, 117, 2479-2490). The "bonding" may be direct bonding or may be mediated by a linker. The linker is not particularly limited as long as the linker neither interferes with the action of the alkylating agent nor interferes with the recognition of an immune checkpoint-related gene. Examples thereof can include an amide bond, a phosphodisulfide bond, an ester bond, a coordination bond, and an ether bond. Specific examples of the linker include an indole linker described in International Publication No. WO2005/087762. In this case, the conjugate of the present invention is a conjugate comprising the alkylating agent bonded via the indole linker to the end of pyrrole imidazole polyamide.

The conjugate of the DNA-binding compound and the alkylating agent of the present invention can be used as an alkylating agent specific for an immune checkpoint-related gene. This alkylating agent targets, in combination, genes encoding immune checkpoint molecules and specifically binds to and alkylates the gene regions encoding the immune checkpoint molecules. Specifically, the alkylating agent can target a plurality of genes encoding the immune checkpoint molecules described above.

The alkylating agent comprising the conjugate of the present invention may comprise a carrier or an additive according to the intended purpose, in addition to the conjugate of the present invention. Examples of such a carrier and an additive include water, acetic acid, organic solvents, collagen, polyvinyl alcohol, polyvinylpyrrolidone, carboxyvinyl polymers, carboxymethylcellulose sodium, sodium polyacrylate, sodium alginate, water-soluble dextran, carboxymethyl starch sodium, pectin, methylcellulose, ethylcellulose, xanthan gum, gum arabic, casein, agar, polyethylene glycol, diglycerin, glycerin, propylene glycol, Vaseline, paraffin, stearyl alcohol, stearic acid, human serum albumin, mannitol, sorbitol, lactose, and surfactants. The amount of the conjugate of the present invention used can be appropriately adjusted according to the intended purpose.

Examples of the conjugate of the present invention can include a conjugate given below. This conjugate is a conjugate comprising secoCBI bonded via an indole linker to the end of pyrrole imidazole polyamide. The conjugate is referred to as CCC07-01. The chemical formula is represented by C₉₀H₉₃ClN₃₀O₁₆, and the molecular weight is 1884.71.

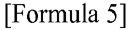

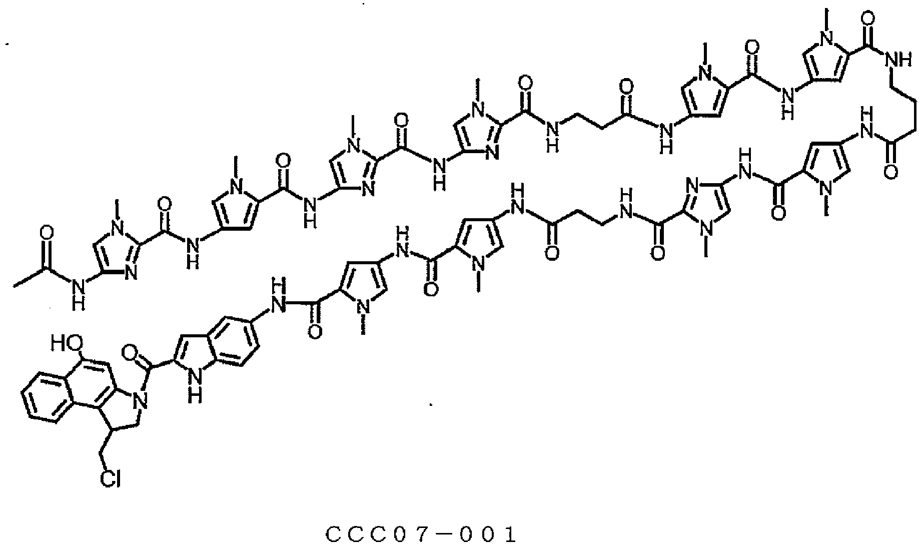

Further examples of the compound of the present invention can include two compounds given below. These conjugates are conjugates comprising secoCBI bonded via an indole linker to the end of pyrrole imidazole polyamide. These conjugates are referred to as CCC07-03 and CCC07-04. The chemical formula of CCC07-03 is represented by C₈₉H₉₂ClN₃₁O₁₆, and the molecular weight is 1887.331. The chemical formula of CCC07-04 is represented by C₈₉H₉₂ClN₃₁O₁₆, and the molecular weight is 1887.331.

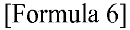

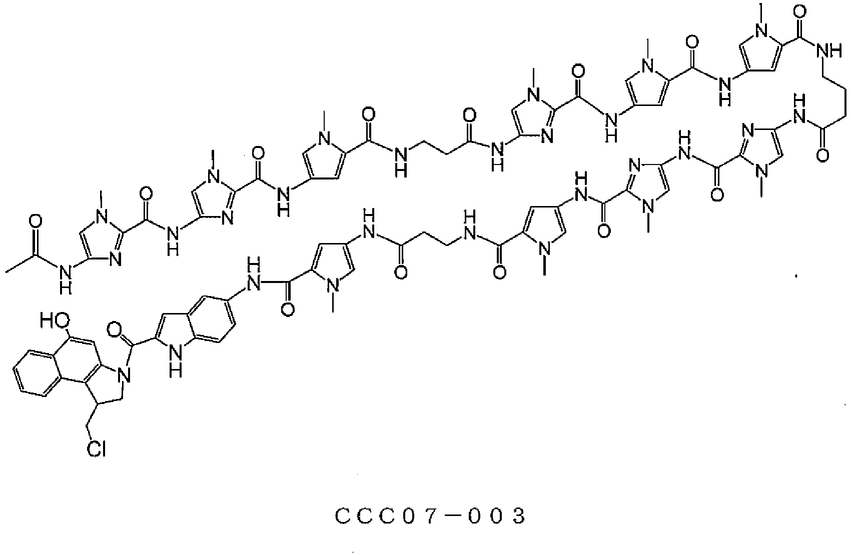

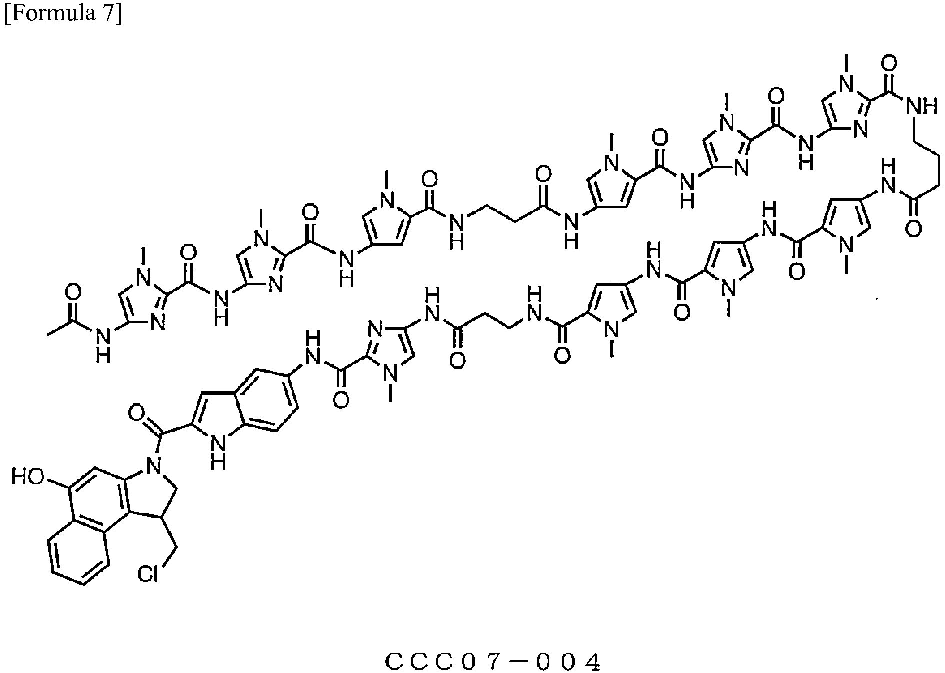

The pharmaceutical composition of the present invention is a composition comprising the conjugate. Various diseases can be treated and prevented by administering the pharmaceutical composition into organisms. The pharmaceutical composition of the present invention can target a disease in every organism, particularly, mammal (e.g., human, rat, rabbit, sheep, pig, cattle, cat, dog, and monkey), which exploits double-stranded DNA in biocontrol. Examples of the disease targeted by the pharmaceutical composition of the present invention include diseases involving a gene mutation, for example, cancer, neurological disease or psychiatric disease, lifestyle-related disease, sleep disorder, disease and infection with strong local symptoms in the dermatological, ophthalmologic, or otolaryngologic field, allergic disease, disease involving cellular senescence, aging, and gastrointestinal disease. Among the target diseases, examples of the cancer can include skin cancer (malignant melanoma, basal cell skin cancer, etc.), brain tumor, head and neck cancer, esophageal cancer, tongue cancer, lung cancer, breast cancer, pancreatic cancer, stomach cancer, cancer of the small intestine or the duodenum, large intestine cancer (colon cancer and rectum cancer), bladder cancer, kidney cancer, liver cancer, prostate cancer, uterus cancer, ovary cancer, thyroid gland cancer, gallbladder cancer, throat cancer, sarcoma (e.g., osteosarcoma, chondrosarcoma, Kaposi's sarcoma, myosarcoma, angiosarcoma, and fibrosarcoma), leukemia (e.g., chronic myeloid leukemia (CML), acute myeloid leukemia (AML), chronic lymphocytic leukemia (CLL), acute lymphatic leukemia (ALL), lymphoma, and multiple myeloma (MM)), childhood solid tumor (brain tumor, neuroblastoma, hepatoblastoma, nephroblastoma, Ewing's sarcoma, etc.), retinoblastoma, and melanoma. Examples of the lifestyle-related disease can include, but are not particularly limited to, hypertension and diabetes mellitus. Examples of the disease and the infection with strong local symptoms in the dermatological, ophthalmologic, or otolaryngologic field can include psoriasis, chronic dermatitis, sinusitis, glaucoma, and retinal degeneration. Examples of the allergic disease can include atopic dermatitis and pollinosis. Examples of the disease involving cellular senescence can include skin wrinkles, sagging, and pigmentation. Examples of the neurological disease or the psychiatric disease can include mania, depression, schizophrenia, autism, bipolar disorder, Alzheimer's disease, sleep disorder, and dementia.

The disease targeted by the pharmaceutical composition of the present invention preferably includes all diseases involving immune checkpoint. The target disease also includes all diseases derived from the immune checkpoint molecules PD-1, PD-L1, and CTLA-4 and their related genes. Examples thereof include malignant melanoma, large intestine cancer, pancreatic cancer, colorectal cancer, thyroid gland cancer, lung cancer, head and neck cancer, esophageal cancer, tongue cancer, lung cancer, breast cancer, pancreatic cancer, stomach cancer, endometrial cancer, myelodysplastic syndrome, adenocarcinomas of the thyroid gland and the colon, and neuroblastoma. The pharmaceutical composition of the present invention acts more effectively on tumor cells rather than normal cells and can treat and prevent a disease by promoting the cross-linking of DNA through alkylation to induce a cell death mechanism based on DNA damage, further silencing an immune checkpoint gene, and killing causative cells of the disease by the inhibition of cell growth and the activation of autoimmune.

The pharmaceutical composition of the present invention may be in any of dosage forms of oral administration and parenteral administration. These dosage forms can be formulated according to a routine method and may contain a pharmaceutically acceptable carrier or additive. Examples of such a carrier and an additive include water, acetic acid, pharmaceutically acceptable organic solvents, collagen, polyvinyl alcohol, polyvinylpyrrolidone, carboxyvinyl polymers, carboxymethylcellulose sodium, sodium polyacrylate, sodium alginate, water-soluble dextran, carboxymethyl starch sodium, pectin, methylcellulose, ethylcellulose, xanthan gum, gum arabic, casein, agar, polyethylene glycol, diglycerin, glycerin, propylene glycol, Vaseline, paraffin, stearyl alcohol, stearic acid, human serum albumin, mannitol, sorbitol, lactose, and surfactants acceptable as pharmaceutical additives.

The additive is selected alone or in appropriate combination from those described above according to the dosage form of the pharmaceutical composition of the present invention. Examples of the dosage form for oral administration include tablets, capsules, fine granules, powders, granules, solutions, syrups, sprays, endermic liniments, eye drops, and preparations for external use. Alternatively, the oral administration may be performed in an appropriate dosage form. Examples of the dosage form for parenteral administration include injections. The injections can be administered systemically or locally by, for example, intravenous injection (e.g., drip infusion), subcutaneous injection, intraperitoneal injection, or intratumoral injection.

For example, for use as a preparation for injection, the pharmaceutical composition of the present invention is dissolved in a solvent (e.g., saline, a buffer solution, a glucose solution, and 0.1% acetic acid), and this solution can be supplemented with an appropriate additive (human serum albumin, PEG, a mannose-modified dendrimer, a cyclodextrin conjugate, etc.) and used. Alternatively, the pharmaceutical composition of the present invention may be freeze-dried for a dosage form that is dissolved before use. For example, a sugar alcohol or a saccharide, such as mannitol or glucose, can be used as an excipient for freeze drying.

The dose of the pharmaceutical composition of the present invention or the compound of the present invention differs depending on age, sex, symptoms, administration route, the number of doses, and the dosage form. The dose, for example, for an adult human (60 kg) is 0.01 to 1000 mg, preferably 0.1 to 100 mg, more preferably 1 to 30 mg, per day. The administration method is appropriately selected according to the age of a patient and symptoms. The dose may be administered, for example, once per few days or in one portion or in two to four divided portions per day.

The pharmaceutical composition of the present invention can be used as an anticancer agent. Examples of the type of the targeted cancer include, but are not limited to, large intestine cancer, pancreatic cancer, colorectal cancer, thyroid gland cancer, lung cancer, neck cancer, endometrial cancer, myelodysplastic syndrome, adenocarcinomas of the thyroid gland and the colon, and neuroblastoma. The anticancer agent targets cancer that is derived from the immune checkpoint gene of PD-1, PD-L1, or CTLA-4 and suppresses tumor immunity, or cancer that suppresses tumor immunity by an immune checkpoint-related gene. The anticancer agent of the present invention may comprise a carrier or a composition according to the intended purpose, as in the pharmaceutical composition and the alkylating agent mentioned above.

The present invention also encompasses a kit. The kit comprises the compound of the present invention as well as a pharmaceutically acceptable carrier or additive, reagents, an auxiliary agent, a dedicated container, other necessary accessories, an instruction manual, etc. The kit of the present invention can also be used as a kit for cancer treatment or a research reagent kit.

### Examples

Hereinafter, the present invention will be described further specifically with reference to Examples. However, the present invention is not limited by these Examples.

### [Example 1] Synthesis of PIP compound CCC07-01 and confirmation of effect

### 1. Overview

The inhibition of immune checkpoint is currently a revolutionary treatment method found also effective for locally progressive and metastatic cancers and considered to produce an additive and/or synergistic effect with chemotherapy, radiotherapy, or vaccine therapy. It is currently desired to inhibit immune checkpoint molecules in combination for improving the response rates of immune checkpoint-inhibiting antibody drugs, to allow immune checkpoint inhibitors to act locally for alleviating adverse reactions, and to prepare a dosage form that can be inexpensively produced from a medical economic standpoint. In this Example, in order to inhibit immune checkpoint molecules in combination, PIP-Indole-secoCBI (CCC07-01) specifically recognizing the consensus nucleotide sequences of the PD-1, PD-L1 and CTLA-4 genes was synthesized by condensing secoCBI that specifically alkylates adenine on the minor groove side of DNA bases. This compound and its analogs were used to conduct (1) an experiment to silence the PD-1 gene, (2) an experiment to silence the PD-L1 gene, (3) an experiment to silence the CTLA-4 gene, (4) an experiment to induce the cell death of cancer cells, (5) an experiment for cellular uptake, and (6) an experiment for accumulating properties in the tumor of cancer cell-transplanted nude mice.

This compound CCC07-01 silenced, at a low concentration, the immune checkpoint molecule genes PD-1, PD-L1 and CTLA-4 in cancer cell lines respectively expressing these genes. The compound also had the ability to suppress tumor growth in a concentration-dependent manner and induced cell death. The uptake of a PIP compound having a log-P value of 1.4 or larger into cultured tumor cells was found to be improved in a log-P value-dependent manner. It was confirmed in a compound having a high log-P value that: the accumulation and storage of the compound in tumor tissues transplanted in mice and their peripheral cells are promoted; and the compound was excreted in 24 hours from normal organs, whereas the compound accumulated in tumor even after 72 hours. The compound CCC07-01 of this study had a log-P value of 3.1 and was presumed to accumulate in tumor cells and peritumoral tissues *in vitro* and *in vivo.*

These results suggest that the present compound specifically alkylates the nucleotide sequence of an immune checkpoint gene and selectively inhibits the expression of the gene, and can suppresses the immune checkpoint gene in cancer cells and a pericancerous microenvironment to induce cell death, thereby activating tumor immunity while inhibiting the immunosuppression mechanism of tumor. Furthermore, the suppression of immune checkpoint mainly in tumor and a tumor microenvironment can also be expected to minimize autoimmune against normal organs. This can overcome the adverse reaction problems of immune checkpoint inhibitors. These results suggest that the compound is very promising as a novel anticancer agent recognizing immune checkpoint genes in combination in a tumor-specific manner and is an anticancer approach effective for various cancers.

### Design of compound

For a PIP compound, a common 9-base sequence that binds to the gene sequences of PD-1, PD-L1, and CTLA-4 was searched for and determined from Human Dec. 2013 (GRCh38/hg38) Assembly. In PD-1, the compound recognizes, as shown in Figure 1, a TGAGGTCAT sequence positioned from intron 1 chromosome 2 (q37): 241855932 to 241855940 and a TGTGGACTA sequence positioned from exon 5 chromosome 2 (q37): 241851257 to 241851265. In PD-L1, the compound recognizes, as shown in Figure 2, a TGTGGTCTT sequence positioned from intron 1 chromosome 9 (p24): 5451659 to 5451667, a TGTGGTCAA sequence positioned from intron 1 chromosome 9 (p24): 5454835 to 5454843, and a TGAGGTCTT sequence positioned from exon 6 chromosome 9 (p24): 5468337 to 5468345. In CTLA-4, the compound recognizes, as shown in Figure 3, a TGTGGACAT sequence positioned from intron 2 Chr2 (q33): 203871035 to 203871043 and a TGAGGTCAT sequence from 3 prime noncoding Chr2 (q33): 203873569 to 203873577. In order to recognize the WGWGGWCWW sequence, the polyamide was designed using a pair of imidazole and pyrrole recognizing guanine and cytosine and a pair of pyrrole and β alanine recognizing adenine and thymine.

### Synthesis of compound

The method for preparing the compound of the present invention is as follows: PIP having a carboxylic acid terminus was dissolved in 100 µL of NMP. To the solution, iPr2NEt (0.5 µL, 2.86 µmol) and PyBOP (1.0 mg, 1.95 µmol) were added. The reaction solution was reacted at room temperature for 2 hours with stirring. Then, the formation of an active form of 1-hydroxybenzotriazole ester was confirmed by HPLC. After the confirmation, NH2-indole seco-CBI (0.6 mg, 1.58 µmol) was added to the reaction vessel, and the mixture was stirred overnight at room temperature in a nitrogen atmosphere. After the reaction, the reaction product was purified by HPLC (0.1% TFA/CH3CN, 30% - 75% linear gradient, 0 to 30 minutes) and freeze-dried to obtain polyamide CCC07-01, the compound of interest, as a white powder (LC-MS m/e C₉₀H₉₃ClN₃₀O₁₆ [M+H]⁺ calcd: 1885.71, found: 1886.40 [M+2H]²⁺ calcd: 943.86, found: 943.75).

In order to identify cells expressing the PD-1, PD-L1, or CTLA-4 gene, the following 29 types of human cell lines were tested. HEK293 (embryonic kidney cells), HeLa (uterine cervical cancer cells), LS180 (large intestine cancer cells), HT-29 (large intestine cancer cells), SW480 (large intestine cancer cells), HCT116 (large intestine cancer cells), PANC-1 (pancreatic cancer cells), GP2d (large intestine cancer cells), MIA PaCa-2 (pancreatic cancer cells), LU99 (lung cancer cells), CW-2 (large intestine cancer cells), COLO-320 (large intestine cancer cells), T84 (large intestine cancer cells), SK-LU-1 (lung cancer cells), AsPC-1 (pancreatic cancer cells), SK-MES-1 (lung cancer cells), SHP-77 (lung cancer cells), PC-9 (lung cancer cells), PK-45H (pancreatic cancer cells), LS513 (pancreatic cancer cells), PK-1 (pancreatic cancer cells), PK-59 (pancreatic cancer cells), NCI-H441 (lung cancer cells), T3M-10 (lung cancer cells), SW1463 (large intestine cancer cells), RKO (large intestine cancer cells), A375 (malignant melanoma cells), A549 (lung cancer cells), and A2058 (malignant melanoma cells) were each cultured for 48 hours in a 12-well plate. The cells were collected, and RNA was extracted therefrom. The extraction employed RNeasy mini kit (Qiagen N.V., CA). Next, cDNA for reverse transcription was prepared with the extracted RNA as a template according to Super Script VILO cDNA Synthesis Kit (Invitrogen Corp., CA). PCR was performed with the cDNA as a template using primers for PD-1, PD-L1, CTLA-4 and control GAPDH. The PCR conditions for PD-1 involved denaturation reaction at 95°C for 2 minutes and then 35 cycles each involving 95°C for 30 seconds, 56°C for 30 seconds, and 72°C for 30 seconds, followed by final elongation reaction at 72°C for 5 minutes. The PCR conditions for PD-L1 involved denaturation reaction at 95°C for 2 minutes and then 25 cycles each involving 95°C for 30 seconds, 60°C for 30 seconds, and 72°C for 30 seconds, followed by final elongation reaction at 72°C for 5 minutes. The PCR conditions for CTLA-4 involved denaturation reaction at 95°C for 2 minutes and then 35 cycles each involving 95°C for 30 seconds, 60°C for 30 seconds, and 72°C for 30 seconds, followed by final elongation reaction at 72°C for 5 minutes. The PCR conditions for GAPDH involved denaturation reaction at 95°C for 2 minutes and then 20 cycles each involving 95°C for 30 seconds, 55°C for 30 seconds, and 72°C for 30 seconds, followed by final elongation reaction at 72°C for 5 minutes. Each PCR product was analyzed by 2% agarose gel electrophoresis. The amount of the PCR product was calculated using a photograph of a band stained with ethidium bromide.

The PCR employed the following primer sets.

### Primers used in PCR

PD-1F: CTG GGC GGT GCT ACA ACT (SEQ ID NO: 15) and PD-1R: ACG AAG CTC TCC GAT GTG TT (SEQ ID NO: 16) were used for PD-1 detection. PD-L1-F2: TGG CAT TTG CTG AAC GCA TTT (SEQ ID NO: 17) and PD-L1-R2: TGC AGC CAG GTC TAA TTG TTT T (SEQ ID NO: 18) were used for PD-L1 detection. CTLA-4F: CTC AGC TGA ACC TGG CTA CC (SEQ ID NO: 19) and CTLA-4R: AGT GGC TTT GCC TGG AGA T (SEQ ID NO: 20) were used for CTLA-4 detection (for RT-PCR). GAPDH-F: CGA CCA CTT TGT CAA GCT CA (SEQ ID NO: 21) and GAPDH-R: AGG GGT CTA CAT GGC AAC TG (SEQ ID NO: 22) were used for the control.

The experimental results are shown in Table 1 with expression indicated by + and no expression indicated by -. PD-1 was expressed in LS180, SW480, GP2d, PC-9, LS513, PK-1, PK-59, NCI-H441, T3M-10, and SW1463. PD-L1 was expressed in SK-MES-1, PC-9, PK-45H, PK-1, PK-59, NCI-H441, T3M-10, SW1463, and RKO. CTLA-4 was expressed in MIA PaCa-2, A375, and A2058.

**[Table 1]**

| | PD-1 | PD-L1 | CTLA-4 |
|---|---|---|---|
| ①HEK293(kidney) | - | - | - |
| ②HeLa(Cervix) | - | - | - |
| ③LS180(Colon) | + | - | - |
| ④HT29(Colon) | - | - | - |
| ⑤SW480(Colon) | + | - | - |
| ⑥HCT116(Colon) | - | - | - |
| ⑦PANC-1(Pancreas) | - | - | - |
| ⑧GP2d(Colon) | + | - | - |
| ⑨MIA-PaCa-2(Pancreas) | - | - | + |
| ⑩LU99(Lung) | - | - | - |
| ⑪CW-2(Colon) | - | - | - |
| ⑫COLO-320(Colon) | - | - | - |
| ⑬T84(Colon) | - | - | - |
| ⑭SK-LU-1(Lung) | - | - | - |
| ⑮AsPC-1(Pancreas) | - | - | - |
| ⑯SK-MES-1(Lung) | - | + | - |
| ⑰SHP-77(Lung) | - | - | - |
| ⑱C-9(Lung) | + | + | - |
| ⑲PK-45H(Pancreas) | - | + | - |
| ⑳LS513(Pancreas) | + | - | - |
| 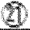 PK-1(Pancreas) | + | + | - |
| 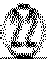 PK-59(Pancreas) | + | + | - |
| 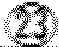 NCI-H441(Lung) | + | + | - |
| 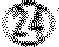 T3M-10(Lung) | + | + | - |
| 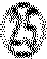 SW1463(Rectum) | + | + | - |
| 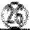 RKO(Colon) | - | + | - |
| 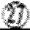 A375(Melanoma) | - | - | + |
| 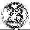 A549(Lung) | - | - | - |
| 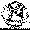 A2058(Melanoma) | - | - | + |

### 2. Cytotoxicity test and 50% inhibitory concentration (IC50) measurement experiment 24 hours after administration to various cells

From the cell lines mentioned above, a large intestine cancer cell line SW480 was used as cells expressing PD-1, a large intestine cancer cell line RKO was used as cells expressing PD-L1, and a malignant melanoma cell line A2058 and a pancreatic cancer cell line MIA PaCa-2 were used as cells expressing CTLA-4. The cells of each line were inoculated at 3000 cells/well to a 96-well microplate and cultured overnight. On the next day, the cells were treated with CCC07-01 having a concentration of 0.1, 1, 10, 30, 100, 300, or 1000 nM (1 µM) for 24 hours. Then, WST-8 reagent was added to each well using Cell counting kit-8, and the mixture was reacted for 2 hours. Then, the cell count was calculated at a wavelength of 450 nm by the colorimetric analysis method using a microplate reader. The method for calculating the cell count involved: 1) obtaining the mean value of a medium alone, 2) subtracting the mean value of the medium from each well, 3) obtaining the mean value of DMSO, 4) dividing the medium mean value-subtracted value by the mean value of DMSO and then multiplying by 100, and 5) obtaining the mean value of each treatment group and a standard deviation, and preparing a graph. IC50 was calculated according to the following expression: IC50 = 10(LOG(A / B) × (50 - C) / (D - C) + LOG(B) wherein A: a higher concentration of two concentrations sandwiching 50%, B: a lower concentration of two concentrations sandwiching 50%, C: the rate of inhibition at B, and D: the rate of inhibition at A.

The cells used in the experiment were RKO (large intestine cancer), SW480 (large intestine cancer), A2058 (malignant melanoma), and MIA PaCa-2 (pancreatic cancer).

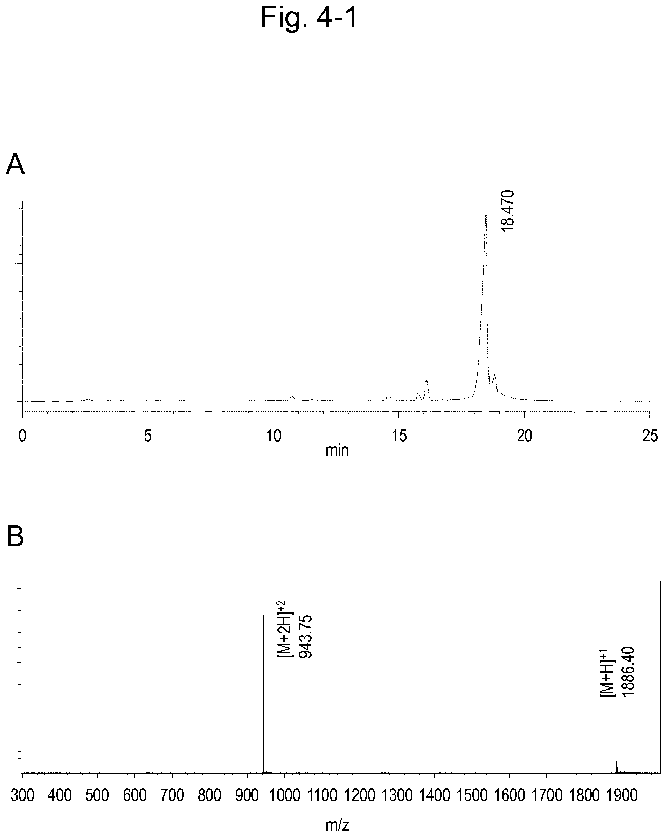

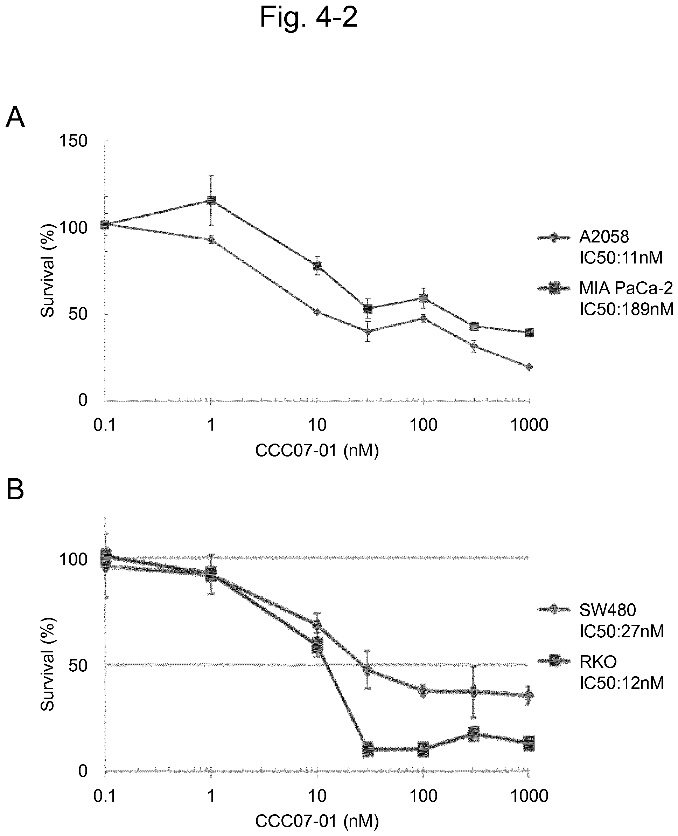

Table 2 shows the experimental results, IC50 of the cells of each line, and the expression of PD-1, PD-L1, and CTLA-4 in their respective cells. As for IC50, CCC07-01 exhibited cytotoxicity to RKO, A2058, and SW480 at a low concentration and exhibited cytotoxicity to MIA PaCa-2 at a slightly higher concentration but in a nanomolar range. Although MIA PaCa-2 expressed only CTLA-4, the expression was lower than that of A2058 also expressing only CTLA-4. The experimental results are shown in Figure 4-1, Figure 4-2 and Table 2. Figure 4-1A shows a chromatogram of HPLC, and Figure 4-1B shows a chromatogram of LC-MS. Figure 4-2A shows the results of the cytotoxicity test by WST assay 24 hours after the administration of CCC07-01 (0.1 nM, 1 nM, 10 nM, 30 nM, 100 nM, 300 nM and 1 µM) to the A2058 and MIA PaCa-2 cells. Concentration-dependent decrease in cell survival rate was seen. Figure 4-2B shows similar results obtained in similar WST assay using the SW480 and RKO cells.

**[Table 2]**

| Cell name | IC50 (nM) | PD-1 expression | PD-L1 expression | CTLA-4 expression |
|---|---|---|---|---|
| RKO | 12 | - | ++ | - |
| SW480 | 27 | ++ | - | - |
| A2058 | 11 | - | - | ++ |
| MIA-PaCa2 | 189 | - | - | + |

### 3. Experiment to silence PD-1 gene in various cancer cells

### Quantitative gene expression analysis by real-time PCR

SW480 cells were cultured in a 6-well plate. On the next day, the cells were treated with 0, 1, 10, or 100 nM CCC07-01 for 6 hours. The cells were collected, and total RNA was extracted therefrom. The extraction employed RNeasy Plus Mini Kit (Qiagen N.V., CA). Next, cDNA was prepared by reverse transcription with the extracted RNA as a template according to Super Script VILO cDNA Synthesis kit (Invitrogen Corp., CA). RT-PCR was performed with the cDNA as a template under the following conditions: the conditions for PD-1 involved denaturation at 95°C and then 33 cycles each involving 95°C for 30 seconds, 56°C for 30 seconds, and 72°C for 30 seconds, followed by elongation reaction at 72°C for 5 minutes. For a control, RT-PCR of GAPDH was performed using the same cDNA as above. The conditions for GAPDH involved denaturation at 95°C and then 22 cycles each involving 95°C for 30 seconds, 55°C for 30 seconds, and 72°C for 30 seconds, followed by elongation reaction at 72°C for 5 minutes.

Each RT-PCR product was electrophoresed on 2% agarose and stained with ethidium bromide. qPCR was further performed. The qPCR conditions involved a holding stage at 95°C for 10 min, a cycling stage at 95°C for 15 sec and at 60°C for 1 min, and a melt curve stage at 95°C for 15 sec, at 60°C for 1 min, and at 95°C for 15 sec. A relative expression level was quantified with GAPDH as a control.

The PCR employed the primer sets described above.

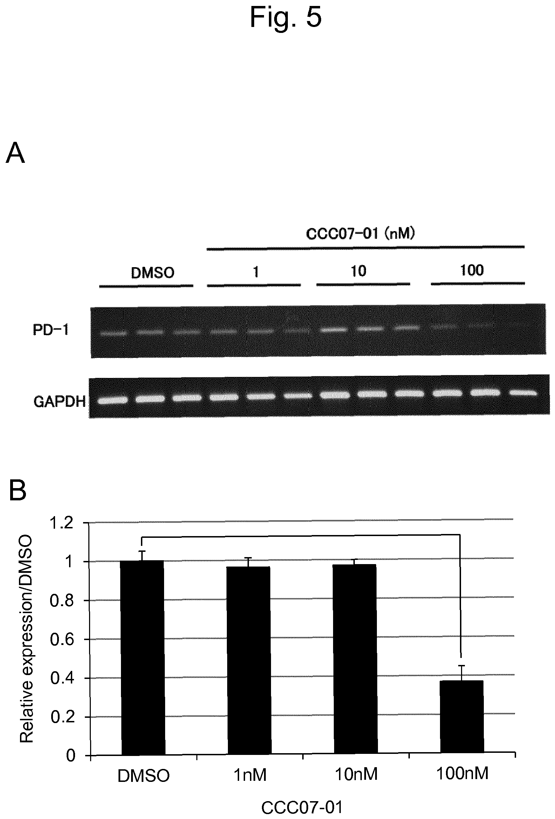

The experimental results are shown in Figure 5. Figure 5A shows the electrophoretic profile, and Figure 5B shows the results of quantification by real-time PCR. As shown in the drawing, the expression of PD-1 was decreased by the administration of the PI polyamide CCC07-01 in a concentration-dependent manner and markedly decreased at 100 nM.

### 4. Experiment to silence PD-L1 gene

### Quantitative gene expression analysis by real-time PCR

RKO cells were cultured in a 6-well plate. On the next day, the cells were treated with 0, 1, 10, or 100 nM CCC07-01 for 24 hours. The cells were collected, and total RNA was extracted therefrom. The extraction employed RNeasy Plus Mini Kit (Qiagen N.V., CA). Next, cDNA was prepared by reverse transcription with the extracted RNA as a template according to Super Script VILO cDNA Synthesis kit (Invitrogen Corp., CA). RT-PCR was performed with the cDNA as a template under the following conditions: the conditions for PD-L1 involved denaturation at 95°C and then 25 cycles each involving 95°C for 30 seconds, 60°C for 30 seconds, and 72°C for 30 seconds, followed by elongation reaction at 72°C for 5 minutes. For a control, RT-PCR of GAPDH was performed using the same cDNA as above. The conditions for GAPDH involved denaturation at 95°C and then 22 cycles each involving 95°C for 30 seconds, 55°C for 30 seconds, and 72°C for 30 seconds, followed by elongation reaction at 72°C for 5 minutes.

Each RT-PCR product was electrophoresed on 2% agarose and then stained with ethidium bromide. qPCR was further performed. The qPCR conditions involved a holding stage at 95°C for 10 min, a cycling stage at 95°C for 15 sec and at 60°C for 1 min, and a melt curve stage at 95°C for 15 sec, at 60°C for 1 min, and at 95°C for 15 sec. A relative expression level was quantified with GAPDH as a control.

The PCR employed the primer sets described above.

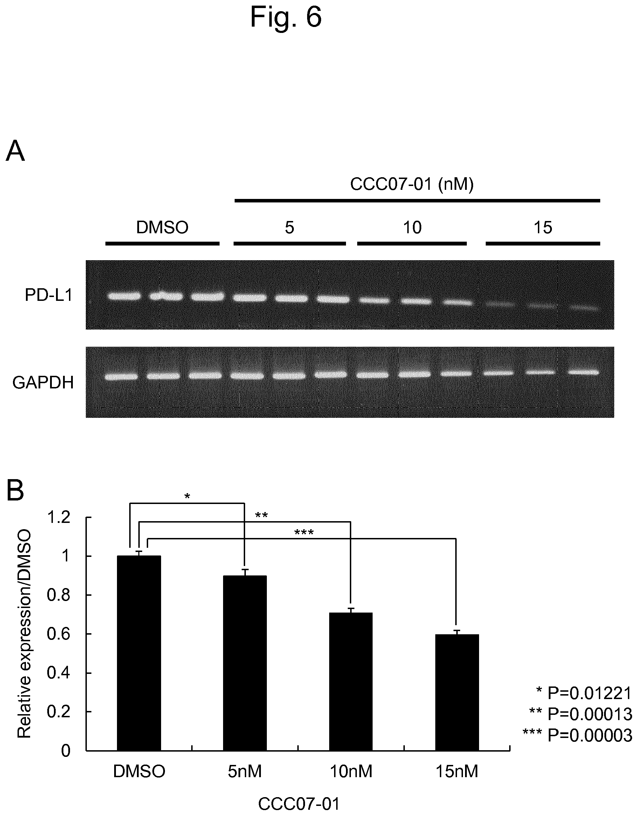

The experimental results are shown in Figure 6. Figure 6A shows the electrophoretic profile, and Figure 6B shows the results of quantification by real-time PCR. As shown in the drawing, the expression of PD-L1 was decreased according to the concentration of CCC07-01 and markedly decreased at 10 nM.

### 5. Experiment to silence CTLA-4 gene

### Semi-quantitative gene expression analysis by RT-PCR

A2058 or MIA PaCa-2 cells were cultured in a 6-well plate. On the next day, the cells were treated with 0, 1, or 10 nM CCC07-01 for 24 hours. The cells were collected, and total RNA was extracted therefrom. The extraction employed RNeasy Plus Mini Kit (Qiagen N.V., CA). Next, cDNA was prepared by reverse transcription with the extracted RNA as a template according to Super Script VILO cDNA Synthesis kit (Invitrogen Corp., CA). RT-PCR was performed with the cDNA as a template under the following conditions: the conditions involved denaturation at 95°C for 2 minutes and then 33 cycles each involving 95°C for 30 seconds, 60°C for 30 seconds, and 72°C for 30 seconds, followed by elongation reaction at 72°C for 5 minutes. For a control, RT-PCR of GAPDH was performed using the same cDNA as above. The conditions for GAPDH involved denaturation at 95°C for 2 minutes and then 22 cycles each involving 95°C for 30 seconds, 55°C for 30 seconds, and 72°C for 30 seconds, followed by elongation reaction at 72°C for 5 minutes. Each PCR product was electrophoresed on 2% agarose and then stained with ethidium bromide. The concentration of the band of the PCR product of CTLA-4 was calculated with the GAPDH band as a control using Image J. The concentrations of the PCR products for each concentration of PIP were compared for quantification.

The PCR employed primer sets of SEQ ID NOs: 19, 20, 21, and 22.

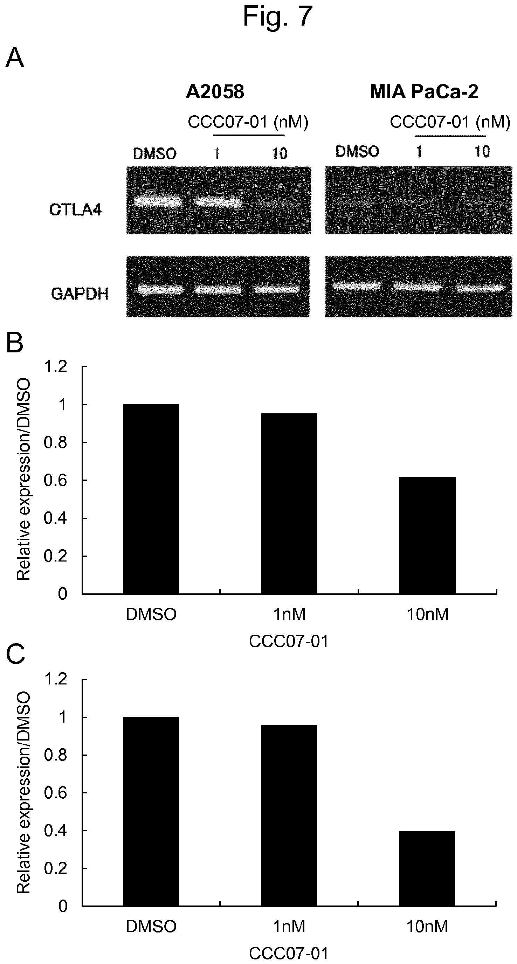

The results are shown in Figure 7. Figure 7A shows the electrophoretic profile, Figure 7B shows the results of quantifying expression in the MIA PaCa-2 cells, and Figure 7C shows the results of quantifying expression in the A2058 cells. As shown in the drawing, the expression of CTLA-4 in the 2058 cells was decreased according to the concentration of CCC07-01 and markedly decreased at 10 nM. The same holds true for MIA PaCa-2.

### 6. Experiment to compare cytotoxicity and 50% inhibitory concentration (IC50) with CBI 48 hours after administration

From the cell lines mentioned above, a large intestine cancer cell line SW480 was used as cells expressing PD-1, and a large intestine cancer cell line RKO was used as cells expressing PD-L1. An experiment was conducted to compare toxicity and IC50 between the PIP-drug conjugate compound CCC07-01 and the alkylating agent CBI used as a drug conjugate therein. The cells of each line were inoculated at 3000 cells/well to a 96-well microplate and cultured overnight. On the next day, the cells were treated with the PIP-CBI conjugate (CCC07-01) or CBI having a concentration of 0.1, 1, 10, 30, 100, 300, or 1000 nM (1 µM) for 48 hours. Then, WST-8 reagent was added to each well using Cell counting kit-8, and the mixture was reacted for 2 hours. Then, the cell count was calculated at a wavelength of 450 nm by the colorimetric analysis method using a microplate reader. The method for calculating the cell count involved: 1) obtaining the mean value of a medium alone, 2) subtracting the mean value of the medium from each well, 3) obtaining the mean value of DMSO, 4) dividing the medium mean value-subtracted value by the mean value of DMSO and then multiplying by 100, and 5) obtaining the mean value of each treatment group and a standard deviation, and preparing a graph. IC50 was calculated according to the following expression: IC50 = 10(LOG(A / B) × (50 - C) / (D - C) + LOG(B) wherein A: a higher concentration of two concentrations sandwiching 50%, B: a lower concentration of two concentrations sandwiching 50%, C: the rate of inhibition at B, and D: the rate of inhibition at A.

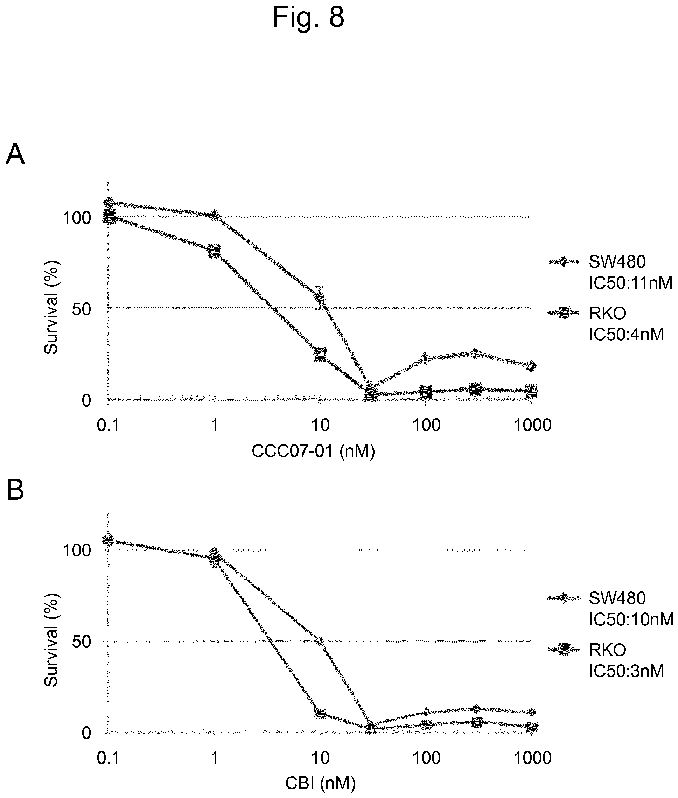

The experimental results are shown in Figure 8. Cytotoxicity to the cells of each line after the administration of CCC07-01 or CBI is shown in the graph. Figure 8A shows the results about CCC07-01 (48 hours), and Figure 8B shows the results about CBI (48 hours). As shown in the drawing, CCC07-01 exhibited IC50 equivalent to that of CBI with 11 nM and 10 nM, respectively, in the SW480 cells and 4 nM and 3 nM, respectively, in the RKO cells.

### 7. Experiment for cellular uptake

SW480 and LS180 were each inoculated at 1 × 10⁵ cells/well, incubated overnight, and treated with each of Im00 to Im05 having a concentration of 1 µM for 2 hours (for Im00 to Im05, see "9. Experiment to measure logP value of compound" described later; Im00, Im01, Im02, Im03, Im04, and Im05 had higher lipid solubility in this order). For a control, the cells were fixed in methanol for 30 minutes using FITC and DMSO (dimethyl sulfoxide) and enclosed using VECTASHIELD. Then, the fluorescent image of each sample was observed under a confocal microscope.

### Cellular uptake

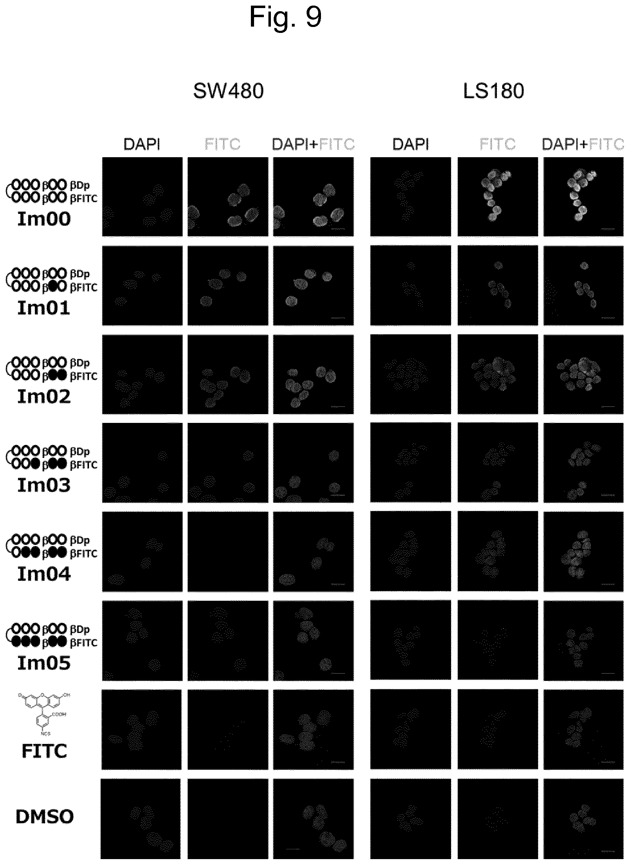

The experimental results are shown in Figure 9. Figure 9 shows the results of DAPI staining, the results of FITC staining, and merged results. Figure 9 shows that the cells were more strongly stained with FITC in the order of Im00, Im01, Im02, Im03, Im04, and Im05. As shown in the drawing, uptake into the cancer cells of both the cell lines was higher for PI polyamide having higher lipid solubility as compared with water-soluble PI polyamide and tended to be poorer with increase in water solubility of the PI polyamide.

### 8. Experiment for accumulating properties in tumor of cancer cell-transplanted nude mouse

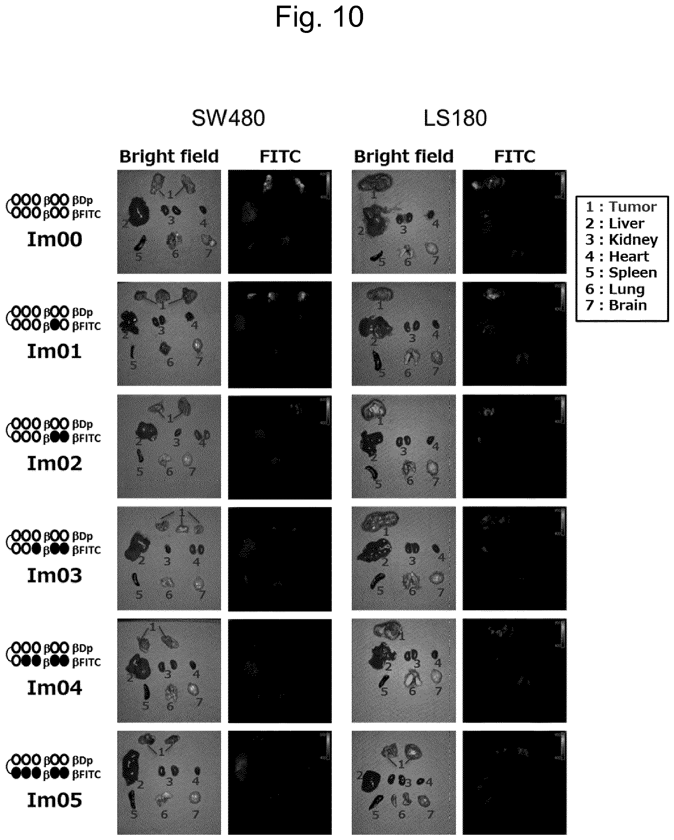

5 × 10⁶ cells of SW480 or 3 × 10⁶ cells of LS180 were subcutaneously transplanted to each nude mouse (BALB/c nu/nu). After the transplantation, Im00 to Im05 were each administered at a concentration of 1.0 mg/kg from the tail vein when tumorigenesis was able to be confirmed. 72 hours after the administration, the mouse was subjected to autopsy, and the fluorescence of a tumor fragment and normal organs was observed by *in vivo* imaging. The experimental results are shown in Figure 10. In Figure 10, "Bright field" depicts the positions of 1: tumor, 2: liver, 3: kidney, 4: heart, 5: spleen, 6: lung, and 7: brain harvested from the mouse, and "FITC" depicts FITC staining. Figure 10 shows that the tumor cells were more strongly stained with FITC in the order of Im00, Im01, Im02, Im03, Im04, and Im05. As shown in the drawing, uptake into the tumor was higher for PI polyamide having higher lipid solubility, as in the cellular uptake. The uptake of water-soluble PI polyamide into the tumor was not observed in the xenograft models of SW480 by *in vivo* imaging.

### 9. Experiment to measure log-P value of compound

### Method for calculating log-P

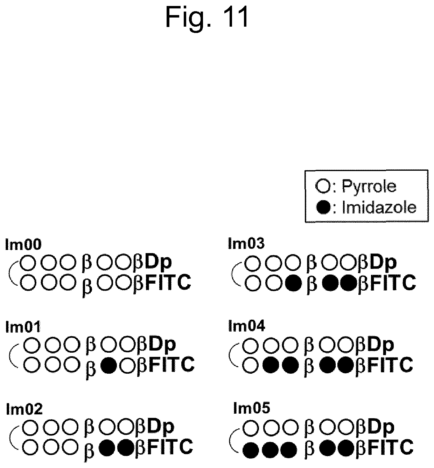

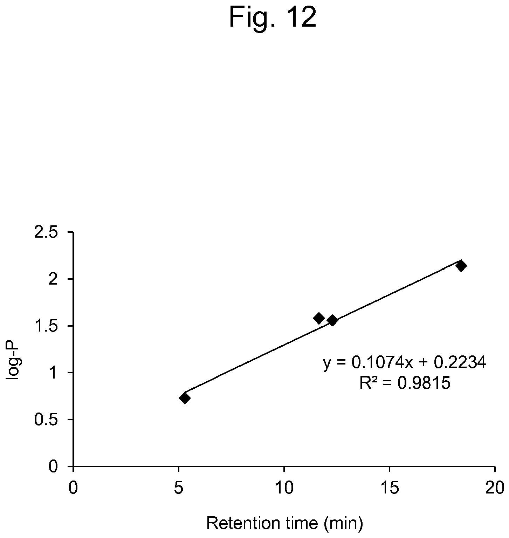

Six types of FITC-added PI polyamides (Im00 to Im05) were analyzed for log-P (octanol/water partition coefficient) by HPLC. The structures of Im00 to Im05 are shown in Figure 11. In Figure 11, "β" represents β alanine, and "Dp" represents N,N-dimethyl-1,3-propanediamine. A linker exists at the left end of the drawing showing each structure, and a U-shaped conformation is taken as a whole. The retention times of 4 types of compounds (ethyl acetate, benzonitrile, acetophenone, and indole) having known log-P were measured in the presence of a mixed solvent of 0.1% aqueous acetic acid solution (75%) and acetonitrile (25%) by HPLC using isocratic elution. A calibration curve was determined from the retention times and the log-P values of these compounds. The calibration curve is shown in Figure 12. The 6 types of PI polyamides (Im00 to Im05) were examined for their retention times by HPLC under the same conditions as above. Their respective log-P values were calculated from the calibration curve. The calculated values are shown in Table 3. These results suggested that a polyamide having a higher log-P value, i.e., higher lipid solubility, has higher tumor accumulating properties.

**[Table 3]**

| | retention time | logP_{ow} |
|---|---|---|
| Ethyl acetate | 5.29 | 0.73 |
| Benzonitrile | 12.30 | 1.56 |
| Acetophenone | 11.65 | 1.58 |
| Indole | 18.40 | 2.14 |
| Im00 | 12.87 | 1.61 |
| Im01 | 11.73 | 1.48 |
| Im02 | 11.40 | 1.45 |
| Im03 | 10.97 | 1.40 |
| Im04 | 10.48 | 1.35 |
| Im05 | 8.86 | 1.18 |

The polyamides were further analyzed for log-P (octanol/water partition coefficient) by HPLC. The retention times of 2 types of compounds (compound names) having a known log-P value and the polyamides were measured in the presence of a mixed solvent of 0.1% aqueous acetic acid solution and acetonitrile by HPLC using isocratic elution. A calibration curve was determined from the retention times and the log-P values of the 2 types of compounds (indole and curcumin). The log-P values of the polyamides were calculated from the curve.

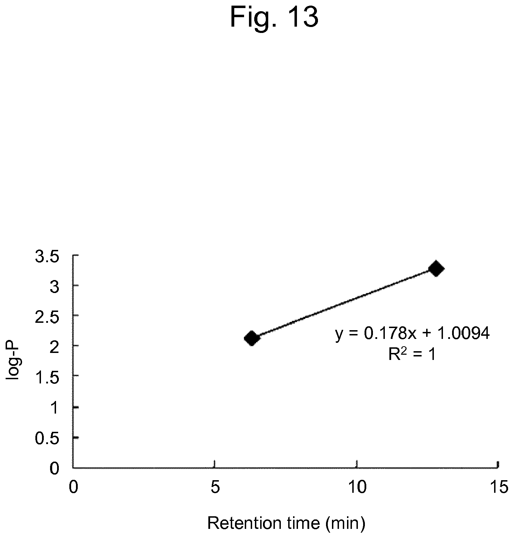

The calibration curve is shown in Figure 13, and the retention times and log-P are shown in Table 4. CCC07-01 exhibited a log-P value of 3 or larger and was found to have high lipid solubility. Thus, this compound was presumed to have high accumulating properties in tumor.

**[Table 4]**

| | retention time (min) | LogP_{ow} |
|---|---|---|
| Indole | 6.35 | 2.14 |
| Curcumin | 12.81 | 3.29 |
| CCC07-001 | 11.90 | 3.13 |

### [Example 2]

1 × 10⁷ cells of human PBMC (Precision Bioservices Inc.) were intravenously transplanted to each NSG mouse (NOD.Cg-Prkdc^{scid}I/2rg^{tm1Wj1}/SzJ; Charles River Laboratories Japan, Inc.) to prepare a humanized NSG mouse. One week after the PBMC transplantation, 1 × 10⁷ RKO cells (ATCC) were subcutaneously transplanted to each humanized NSG mouse. After the transplantation, CCC07-01 was intravenously administered at 2.3 or 9.2 mg/kg to the mouse when tumorigenesis was able to be confirmed. The tumor size was measured over time, and the tumor volume (mm3) was calculated according to the expression: Tumor volume = (Major axis × Minor axis²) / 2. One week after the CCC07-01 administration, the spleen was harvested and homogenized in 2% FBS/PBS to prepare a cell suspension. The spleen cells were stained with a fluorescently labeled anti-human CD4 antibody, anti-human CD8 antibody and anti-human Foxp3 antibody and analyzed for the numbers of cytotoxic T cells (CD8-positive cells) and regulatory T cells (Treg: CD4-positive and Foxp3-positive cells) using a flow cytometer. Statistical processing was performed by the Mann-Whitney U test, and the significance level was set to P < 0.05.

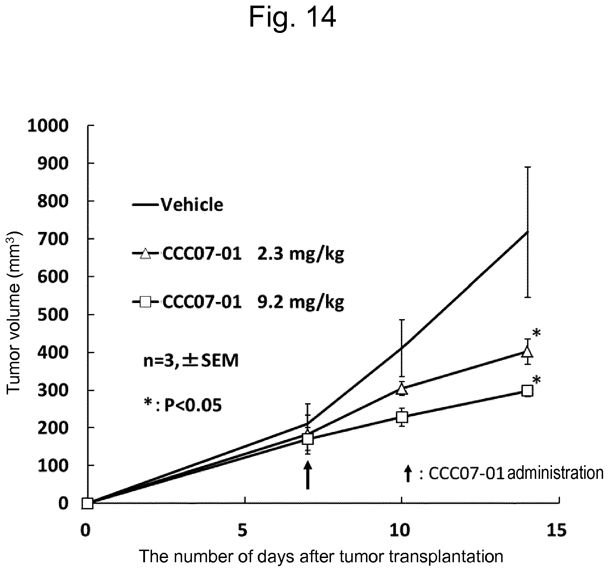

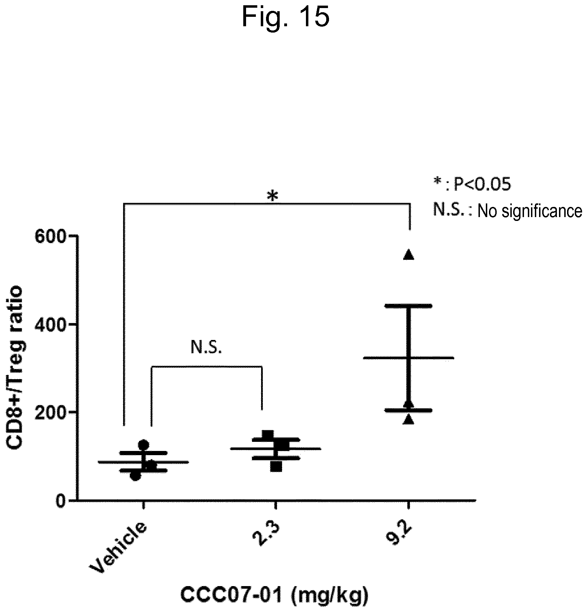

The experimental results are shown in Figures 14 and 15. Figure 14 shows change in tumor volume, and Figure 15 shows the ratio of cytotoxic T cells to Treg in the spleen. CCC07-01 suppressed the growth of the subcutaneously transplanted RKO tumor in a dose-dependent manner. The T/C ratios (%) of the CCC07-01 2.3 and 9.2 mg/kg administration groups were 56.0 and 41.5, respectively. The CD8-positive cell/Treg ratios in the spleen of the vehicle administration group and the CCC07-01 2.3 and 9.2 mg/kg administration groups were 87.9, 116.5, and 323.2, respectively. The significant elevation in CD8-positive cell/Treg ratio seen in the 9.2 mg/kg administration group indicated the possibility that CCC07-01 activates antitumor immune response.

### [Example 3] Synthesis of PIP compounds CCC07-03 and CCC07-04 and confirmation of effect

### Design of additionally synthesized compound

For PIP compound CCC07-03, a common 9-base sequence that binds mainly to the gene sequences of PD-1 and CTLA-4 was searched for and determined from Human Feb. 2009 (GRCh37/hg19) Assembly. CCC07-03 recognizes a TGGTTGCCA sequence positioned from intron 1 chromosome 2 (q37): 242796096 to 242796104 in PD-1. In PD-L1, CCC07-03 recognizes a TGGAAGCCT sequence positioned from intron 1 chromosome 9 (p24): 5455626 to 5455634, a TGGAAGCCA sequence positioned from intron 3 chromosome 9 (p24): 5459365 to 5459373, a TGGATGCCA sequence positioned from intron 5 chromosome 9 (p24): 5466803 to 5466795, and a TGGAATCCA sequence positioned from exon 7 chromosome 9 (p24): 5468657 to 5468665. In CTLA-4, CCC07-03 recognizes a TGGTAGCCA sequence positioned from exon 1 chromosome 2 (q33): 204732717 to 204732709 and a TGGTAGCCT sequence positioned from intron 2 chromosome 2 (q33): 204735791 to 203871043. In order to recognize the WGGWWGCCW sequence, the polyamide was designed using a pair of imidazole and pyrrole recognizing guanine and cytosine and a pair of pyrrole and β alanine recognizing adenine and thymine.

For PIP compound CCC07-04, a common 9-base sequence that binds to the gene sequences of non-human, i.e., mouse Pd-1 (Pdcd1), Pd-11 (Cd274) and Ctla4 was searched for and determined from Mouse Dec. 2011 (GRCm38/mm10) Assembly. CCC07-04 recognizes an ACCAAGCCA sequence positioned from intron 1 chromosome 1 (qD): 94048520 to 94048528 and a TCCTAGCCA sequence positioned from intron 1 chromosome 1 (qD): 94044272 to 94044280 in mouse Pd-1. In Pd-11, CCC07-04 recognizes an ACCATGCCA sequence positioned from exon 1 chromosome 19 (pC1): 29368132 to 29368124. In Ctla-4, CCC07-04 recognizes a TCCAAGCCA sequence positioned from exon 43 prime noncoding region chromosome 1 (qC2): 60915406 to 60915398. In order to recognize the WGGWWGCCW sequence, the polyamide was designed using a pair of imidazole and pyrrole recognizing guanine and cytosine and a pair of pyrrole and β alanine recognizing adenine and thymine.

### Synthesis of compound

The method for preparing the compound of the present invention is as follows: PIP having a carboxylic acid terminus was dissolved in 100 µL of NMP. To the solution, iPr2NEt (0.5 µL, 2.86 µmol) and PyBOP (1.0 mg, 1.95 µmol) were added. The reaction solution was reacted at room temperature for 2 hours with stirring. Then, the formation of an active form of 1-hydroxybenzotriazole ester was confirmed by HPLC. After the confirmation, NH₂-indole seco-CBI (0.6 mg, 1.58 µmol) was added to the reaction vessel, and the mixture was stirred overnight at room temperature in a nitrogen atmosphere. After the reaction, the reaction product was purified by HPLC (0.1% TFA/CH₃CN, 30% - 75% linear gradient, 0 to 30 minutes) and freeze-dried to obtain polyamide PD-1/PD-L1, the compound of interest, as a white powder (LC-MS m/z C₉₀H₉₃ClN₃₀O₁₆ [M+H]⁺ calcd: 1885.71, found: 1886.40 [M+2H]²⁺ calcd: 943.86, found: 943.75).

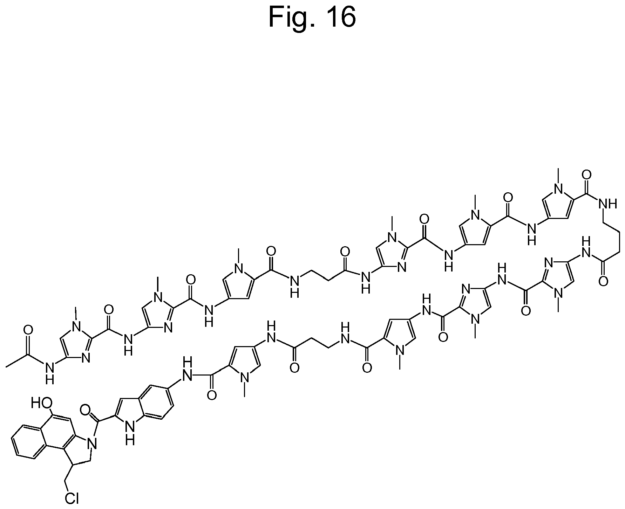

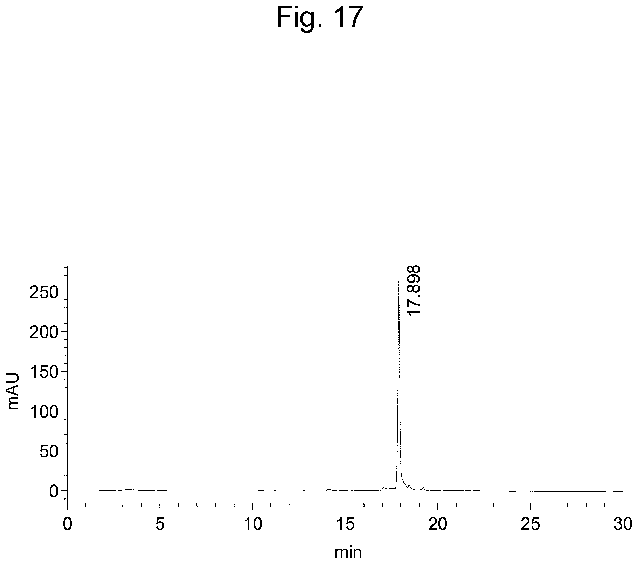

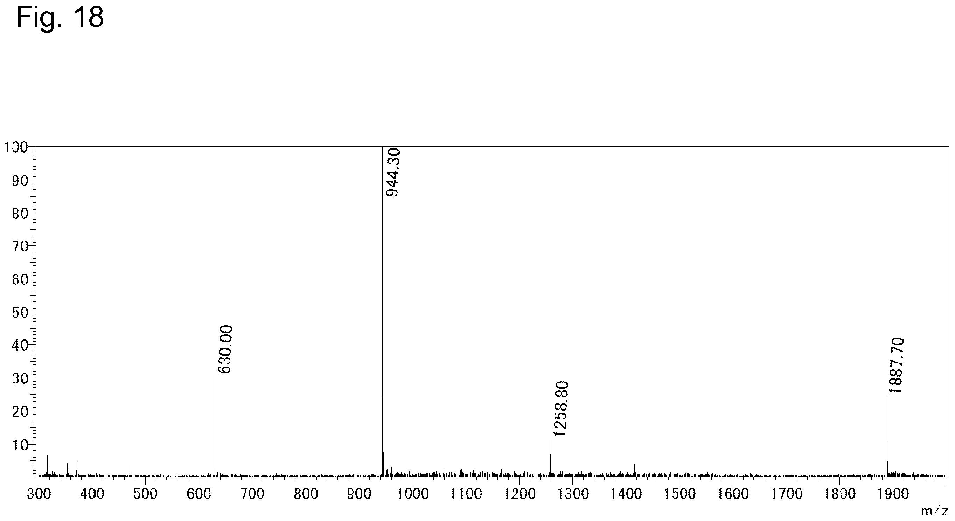

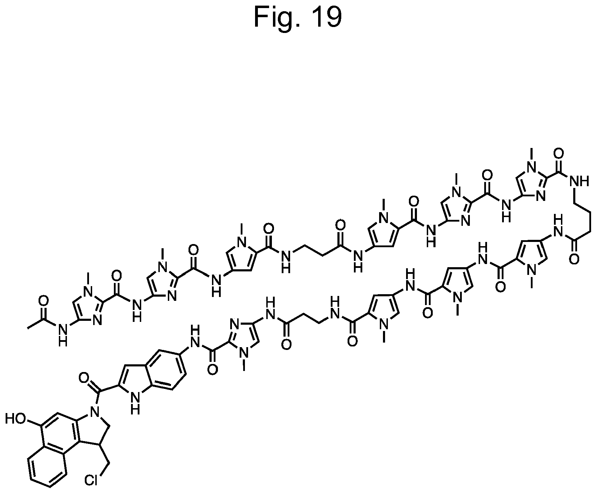

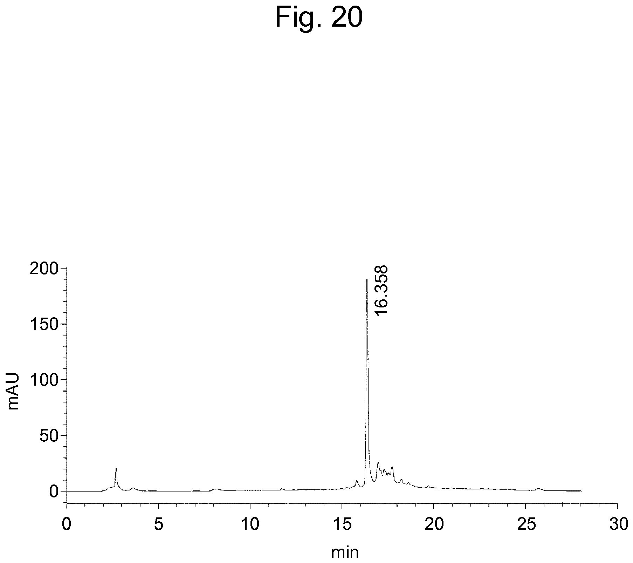

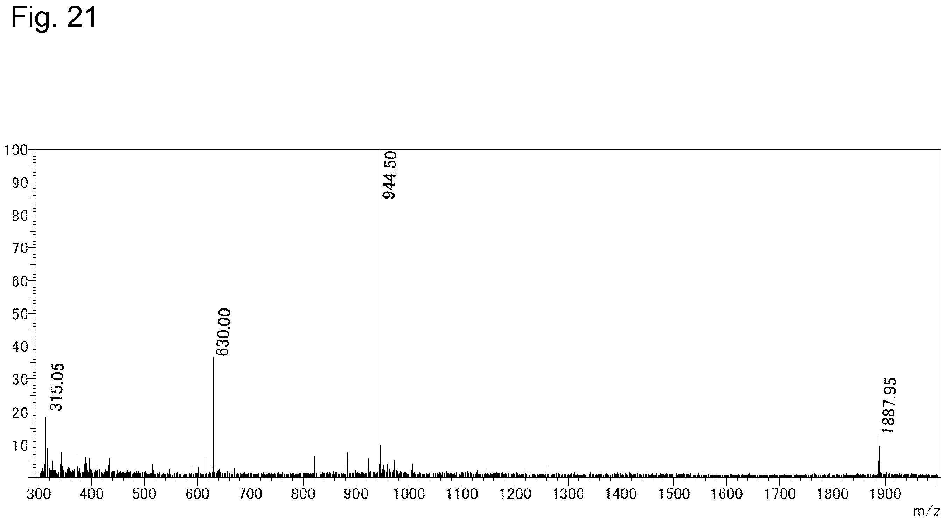

Figure 16 shows the structural formula of CCC07-03, Figure 17 shows its HPLC analysis data, and Figure 18 shows its LC-MC analysis data. Figure 19 shows the structural formula of CCC07-04, Figure 20 shows its HPLC analysis data, and Figure 21 shows its LC-MC analysis data.

### Human gene primers used in PCR

PD-1F: CTG GGC GGT GCT ACA ACT (SEQ ID NO: 15) and PD-1R: ACG AAG CTC TCC GAT GTG TT (SEQ ID NO: 16) were used for PD-1 detection. PD-L1-F2: TGG CAT TTG CTG AAC GCA TTT (SEQ ID NO: 17) and PD-L1-R2: TGC AGC CAG GTC TAA TTG TTT T (SEQ ID NO: 18) were used for PD-L1 detection. CTLA-4F: CTC AGC TGA ACC TGG CTA CC (SEQ ID NO: 19) and CTLA-4R: AGT GGC TTT GCC TGG AGA T (SEQ ID NO: 20) were used for CTLA-4 detection (for RT-PCR). GAPDH-F: CGA CCA CTT TGT CAA GCT CA (SEQ ID NO: 21) and GAPDH-R: AGG GGT CTA CAT GGC AAC TG (SEQ ID NO: 22) were used for a control.

### Experiment to silence two genes by real-time PCR

### Experiment to silence PD-L1 gene

RKO cells were cultured at 1 x 10⁵ cells/well in a 6-well plate. On the next day, the cells were treated with 0, 5, 50, or 500 nM CCC07-03 for 6 hours, 24 hours, or 48 hours. The cells were recovered, and total RNA was extracted therefrom. The extraction employed AllPrep DNA/RNA Mini Kit (Qiagen N.V., CA). Next, cDNA was prepared by reverse transcription with the extracted RNA as a template using Super Script VILO(TM) Master Mix (Invitrogen Corp., CA) and subjected to qPCR. The qPCR conditions involved a holding stage at 50°C for 2 min and 95°C for 10 min, a cycling stage (40 cycles) at 95°C for 15 sec and at 60°C for 1 min, and a melt curve stage at 95°C for 15 sec, at 60°C for 1 min, at 95°C for 30 sec, and at 60°C for 15 sec. A relative expression level was quantified with GAPDH as a control. The PCR of PD-L1 employed the following primer set.

### Primers

### PD-L1

Forward: TGG CAT TTG CTG AAC GCA TTT (SEQ ID NO: 17)
Reverse: TGC AGC CAG GTC TAA TTG TTT T (SEQ ID NO: 18)

### GAPDH

Forward: CGA CCA CTT TGT CAA GCT CA (SEQ ID NO: 21)
Reverse: AGG GGT CTA CAT GGC AAC TG (SEQ ID NO: 22)

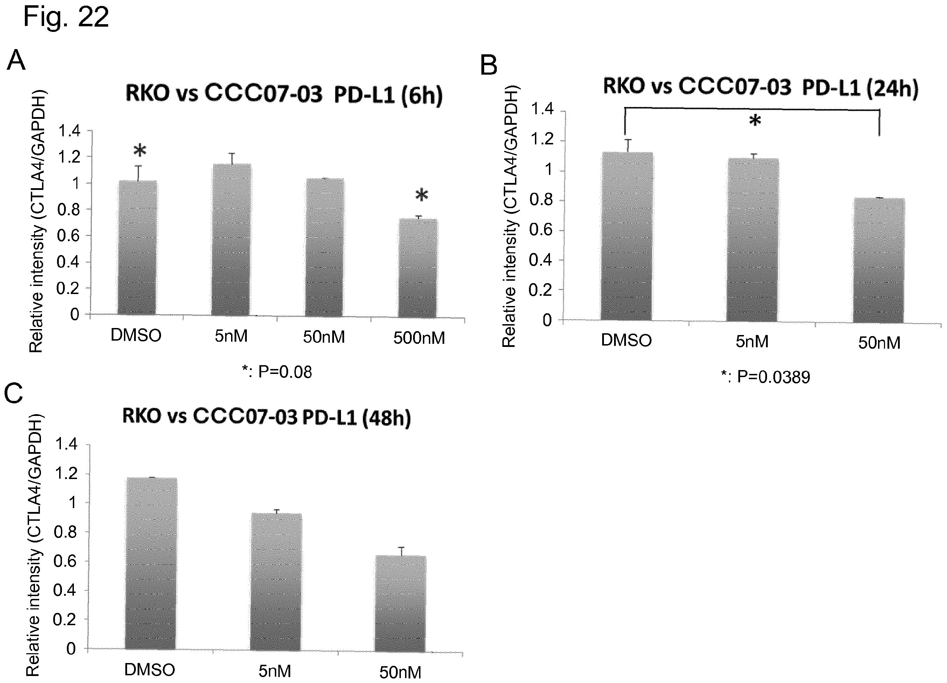

The results of quantifying expression after the administration of CCC07-03 in the RKO cells expressing PD-L1 are shown in Figures 22A, 22B, and 22C. As shown in Figure 22A, the expression of PD-L1 tended to be decreased by the treatment at 500 nM for 6 hours, as compared with the control. As shown in Figure 22B, the expression of PD-L1 was statistically significantly decreased by the administration of CCC07-03 at a concentration of 50 nM in the RKO cells 24 hours after the administration. As shown in Figure 22C, the expression of PD-L1 was decreased according to the concentration of CCC07-03 in the RKO cells 48 hours after the administration.

### Experiment to silence CTLA4 gene

A2058 cells were cultured at 1 x 10⁵ cells/well in a 6-well plate. On the next day, the cells were treated with 0, 5, 50, or 500 nM CCC07-03 for 6 hours, 24 hours, or 48 hours. The cells were recovered, and total RNA was extracted therefrom. The extraction employed AllPrep DNA/RNA Mini Kit (Qiagen N.V., CA). Next, cDNA was prepared by reverse transcription with the extracted RNA as a template using Super Script VILO(TM) Master Mix (Invitrogen Corp., CA) and subjected to qPCR. The qPCR conditions involved a holding stage at 50°C for 2 min and 95°C for 10 min, a cycling stage (40 cycles) at 95°C for 15 sec and at 60°C for 1 min, and a melt curve stage at 95°C for 15 sec, at 60°C for 1 min, at 95°C for 30 sec, and at 60°C for 15 sec. A relative expression level was quantified with GAPDH (primers: SEQ ID NOs: 21 and 22) as a control. The PCR of CTLA4 employed the following primer set.

### Primers

Forward: CTC AGC TGA ACC TGG CTA CC (SEQ ID NO: 19)
Reverse: AGT GGC TTT GCC TGG AGA T (SEQ ID NO: 20)

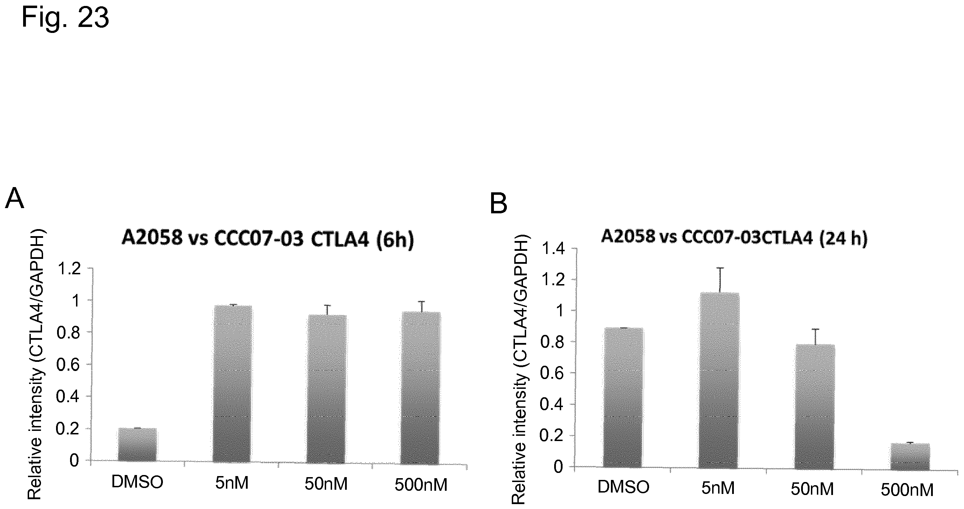

The results of quantifying expression after the administration of CCC07-03 in the A2058 cells expressing CTLA-4 are shown in Figures 23A and 23B. As shown in Figure 23A, the expression of CTLA-4 was elevated by the treatment for 6 hours, as compared with the control. However, as shown in Figure 23B, the expression of CTLA-4 was decreased according to the concentration of CCC07-03 in the A2058 cells 24 hours after the administration and markedly decreased by the treatment at a concentration of 500 nM for 24 hours.

Study on CTLA4 gene silencing by negative control polyamide

A2058 cells were cultured at 1 x 10⁵ cells/well in a 6-well plate. On the next day, the cells were treated with 0, 5, 50, or 500 nM CCC07-04 for 6 hours, 24 hours, or 48 hours. The cells were recovered, and total RNA was extracted therefrom. The extraction employed AllPrep DNA/RNA Mini Kit (Qiagen N.V., CA). Next, cDNA was prepared by reverse transcription with the extracted RNA as a template using Super Script VILO(TM) Master Mix (Invitrogen Corp., CA) and subjected to qPCR. The qPCR conditions involved a holding stage at 50°C for 2 min and 95°C for 10 min, a cycling stage (40 cycles) at 95°C for 15 sec and at 60°C for 1 min, and a melt curve stage at 95°C for 15 sec, at 60°C for 1 min, at 95°C for 30 sec, and at 60°C for 15 sec. A relative expression level was quantified with GAPDH (primers: SEQ ID NOs: 21 and 22) as a control. The PCR of CTLA4 employed the following primer set.

### Primers

Forward: CTC AGC TGA ACC TGG CTA CC (SEQ ID NO: 19)
Reverse: AGT GGC TTT GCC TGG AGA T (SEQ ID NO: 20)

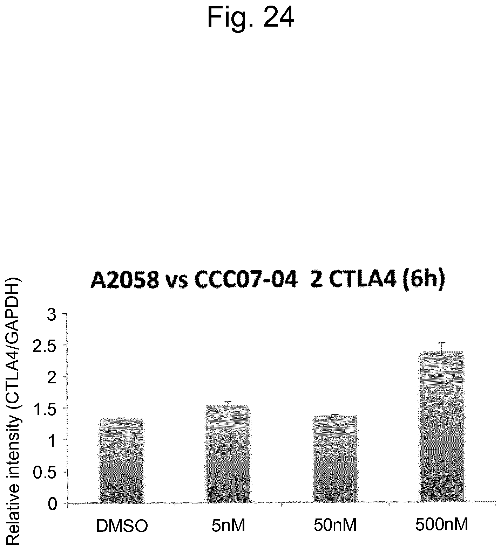

The results of quantifying expression after the administration of CCC07-04 in the A2058 cells expressing CTLA-4 are shown in Figure 24. As shown in Figure 24, the expression of CTLA-4 was elevated by the treatment for 6 hours, as compared with the control. All the cells floated 24 hours or later after the administration, suggesting that cell death was induced. Thus, the measurement was impossible.

### Experiment to silence mouse Pd-11 gene

B16F1 cells were cultured at 1 x 10⁵ cells/well in a 6-well plate. On the next day, the cells were treated with 0, 100, 200, 300, 400, or 500 nM CCC07-04 for 6 hours, 24 hours, or 48 hours. The cells were recovered, and total RNA was extracted therefrom. The extraction employed AllPrep DNA/RNA Mini Kit (Qiagen N.V., CA). Next, cDNA was prepared by reverse transcription with the extracted RNA as a template using Super Script VILO(TM) Master Mix (Invitrogen Corp., CA) and subjected to qPCR. The qPCR conditions involved a holding stage at 50°C for 2 min and 95°C for 10 min, a cycling stage (40 cycles) at 95°C for 15 sec and at 60°C for 1 min, and a melt curve stage at 95°C for 15 sec, at 60°C for 1 min, at 95°C for 30 sec, and at 60°C for 15 sec. A relative expression level was quantified with β-tubulin as a control. The PCR of Pd-11 employed the following primer set.

### Primers

### Pd-11

Forward: TGC TGC ATA ATC AGC TAC GG (SEQ ID NO: 23)
Reverse: GCT GGT CAC ATT GAG AAG CA (SEQ ID NO: 24)

### β-Tubulin

Forward: GCC ACT ACA CCA TTG GCA AG (SEQ ID NO: 25)
Reverse :GTG GAA AAC CAA GAA GCC CT (SEQ ID NO: 26)

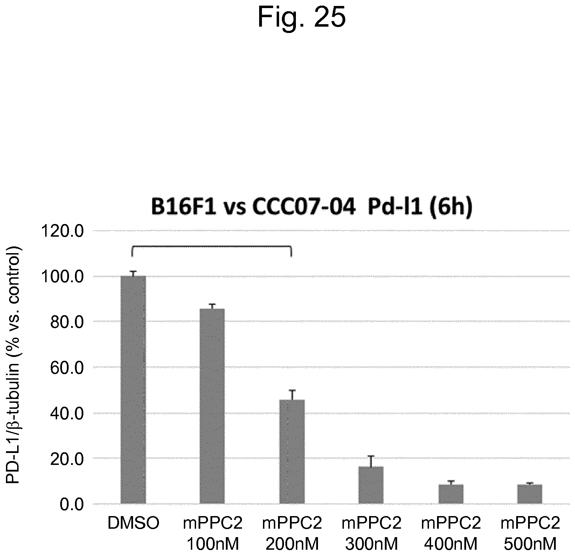

The results of quantifying expression after the administration of CCC07-04 in the mouse B16F1 cells expressing Pd-11 are shown in Figure 25. As shown in Figure 25, the expression of Pd-11 was statistically significantly decreased according to the concentration of CCC07-04 in the B16F1 cells 6 hours after the administration. All the mouse B16F1 cells floated 24 hours or later after the administration of CCC07-04, suggesting that cell death was induced. Thus, the measurement was impossible.

CCC07-04 was designed and synthesized as a compound recognizing mouse Pd-1, Pd-11, and Ctla4 as a surrogate for humans. Although CCC07-04 was confirmed to silence the Pd-11 gene, its effect on Pd-1 or Ctla4 is still unknown because mouse cell lines highly expressing Pd-1 or Ctla4 have not yet been able to be analyzed. CCC07-04 silenced at least the Pd-11 gene in the mouse cell line and has not yet been able to be confirmed to silence the Pd-1, Pd-11, and Ctla4 genes in human cell lines.

### [Example 4] Experiment for long-term accumulating properties in tumor of cancer cell-transplanted nude mouse

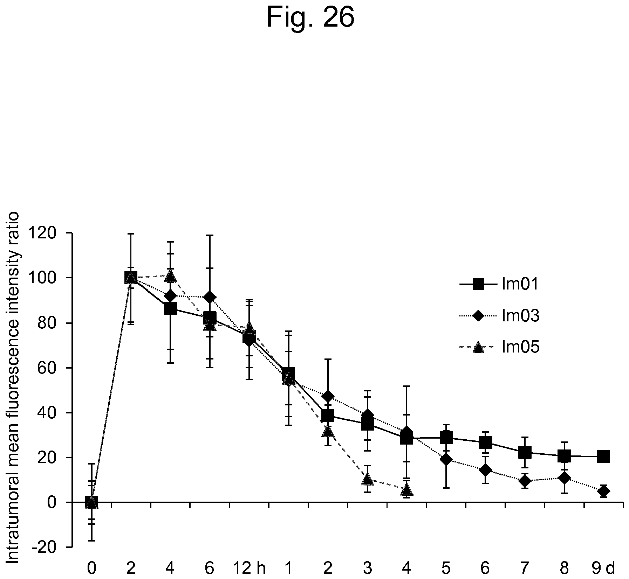

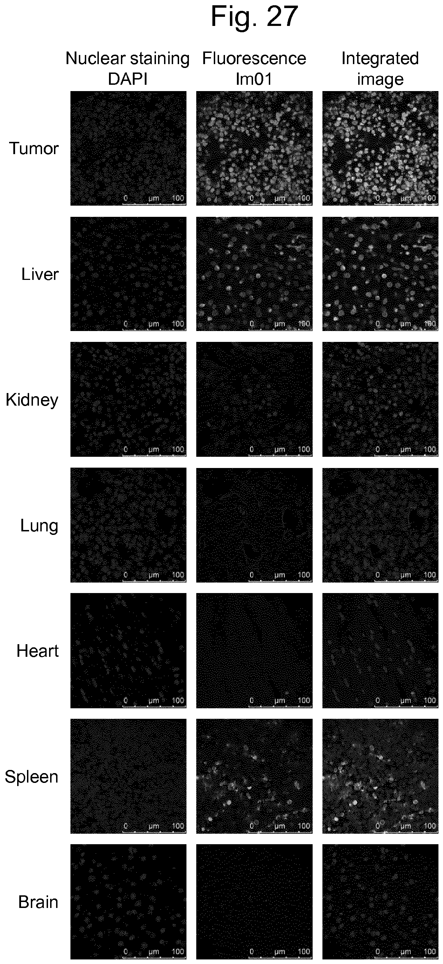

3 × 10⁶ LS180 human large intestine cancer cells were subcutaneously transplanted to each nude mouse (BALB/c nu/nu). After the transplantation, fluorescently FITC-labeled Im01, Im03, and Im05 were each administered at a concentration of 1.0 mg/kg from the tail vein of three tumor-transplanted mice when the tumor diameter reached 1 cm after confirmation of tumorigenesis. The fluorescence intensity of the subcutaneously transplanted tumor site and the fluorescence intensity of a non-transplanted site were measured by *in vivo* imaging immediately before the administration (0 hours) and 2, 4, 6, and 12 hours and 1, 2, 3, 4, 5, 6, 7, 8, and 9 days after the administration, and the ratio therebetween was determined. Since the maximum fluorescence intensity was obtained 2 hours after the administration, this fluorescence intensity was defined as 100. The fluorescence intensity was determined by percentage at each time after the administration. The mean value and SD value of the three mice at each time after the administration are shown in a line graph form in Figure 26 as to each fluorescent compound. The mice were further subjected to autopsy 9 days (216 hours) after the administration. Frozen sections of tumor fragments and tissues of major normal organs (liver, kidney, lung, heart, spleen, and brain) were prepared, and the fluorescence of these sections was observed under a confocal microscope at a magnification of ×400. For the observation, DAPI staining was performed beforehand to fluorescently stain the nuclei in the cells. The coexistence of the nuclear fluorescence and the fluorescently labeled PI polyamide compounds was examined by comparing their respective fluorescent observation with integrated images prepared from the observation images. The experimental results (fluorescent observation (×400) at the divided faces of the tissues 9 days (216 hours) after the Im01 administration in the LS180 xenograft mice) are shown in Figure 27.

### Results

For highly lipid-soluble Im01, strong fluorescence intensity was observed at the tumor site, suggesting the compound was stored in tumor even 9 days after the administration. For Im03, strong fluorescence intensity that indicated intratumoral storage was observed at the tumor site, but was decreased to almost the background level at 9 days after the administration. For Im05, strong fluorescence intensity that indicated intratumoral storage was observed at the tumor site, but was decreased to almost the background level at 4 days after the administration. Thus, the polyamide compounds exhibited the property of being taken up into tumor, accumulated, and stored for a long period. Particularly, high tumor storage was observed in the highly lipid-soluble polyamide compound.

The mice given Im01 that exhibited the long-term storage of fluorescence were euthanized at day 9, and fluorescence intensity was observed in tumor and major organs. As a result, strong fluorescence intensity was shown in the nuclei of the cells. Particularly, strong fluorescence was observed at the tumor site in agreement with the nuclei. In the major organs, a weak tendency in the nuclei was observed in the liver and the spleen, and a weaker tendency in the nuclei was also observed in the kidney.

From the observation results described above, the lipid-soluble polyamide compound was confirmed to have the property of being taken up into tumor, accumulated, and stored for a long period.

### Industrial Applicability

The conjugate of the present invention can be used as an active ingredient for a pharmaceutical composition such as an anticancer agent.

### Free Text of Sequence Listing

SEQ ID NOs: 15 to 26 - Primers

## Claims

1. A conjugate of a DNA-binding compound and an alkylating agent, wherein the DNA-binding compound specifically binds to a gene region encoding an immune checkpoint molecule or a related molecule thereof.

2. The conjugate according to claim 1, wherein the conjugate accumulates in tumor and a peritumoral microenvironment and specifically suppresses the immune checkpoint molecule or the related molecule thereof in the tumor.

3. The conjugate according to claim 1 or 2, wherein the immune checkpoint molecule is selected from the group consisting of PD-1, PD-L1, CTLA-4, CD80, CD86, PD-L2, and CD28, and the molecule related to the immune checkpoint molecule is at least one or more members selected from the group consisting of LAG3, TIGIT, IFNγ, IFN-I, IDO, KIR2DL-1, KIR2DL-2, KIR2DL-3, KIR2DS-1, KIR2DA-2, B7-H3 (CD276), TIM-3, VISTA, B7RP1, ICOS, B7-H4, HVEM, BTLA, CD137, CD70, OX40, CD40, CD137L, CD70L, OX40L, CD40L, GAL9, A2eR, TGFβ, IL1, IL6, IL10, IL12 and IL18, and immune-related molecules registered in gene databases.

4. The conjugate according to any one of claims 1 to 3, wherein the DNA-binding compound is selected from the group consisting of a bridged nucleic acid, a locked nucleic acid (LNA), PNA, pyrrole imidazole polyamide (PIP), a modified form of pyrrole imidazole polyamide (PIP), a DNA-binding protein, and a DNA-binding protein complex.

5. The conjugate according to any one of claims 1 to 4, wherein the alkylating agent is a compound having a functional group having the ability to alkylate a particular nucleotide sequence of DNA.

6. The conjugate according to claim 5, wherein the alkylating agent is secoCBI.

7. An alkylating agent specific for a gene encoding an immune checkpoint molecule or a related molecule thereof, the alkylating agent comprising a conjugate according to any one of claims 1 to 6.

8. The conjugate according to claim 1, wherein the conjugate is represented by the following formula:

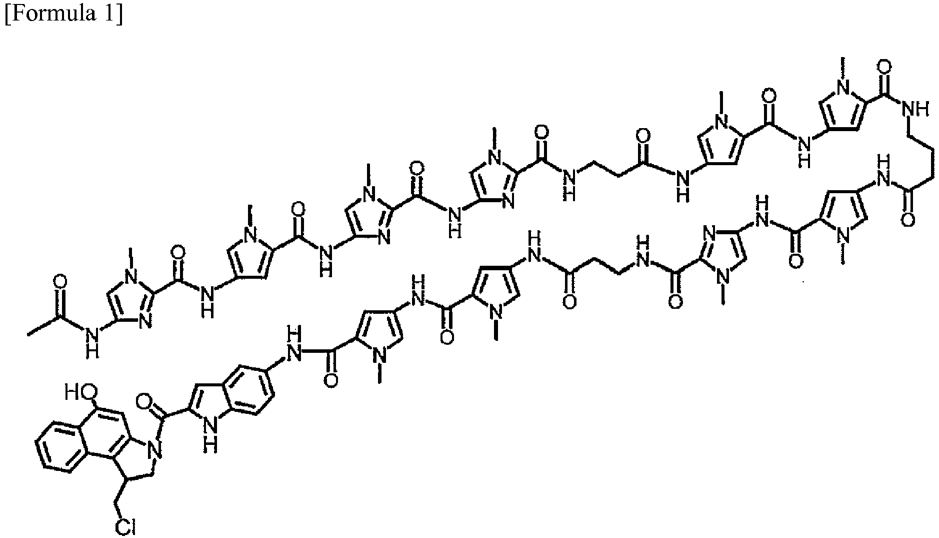

9. The conjugate according to claim 1, wherein the conjugate is represented by the following formula:

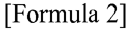

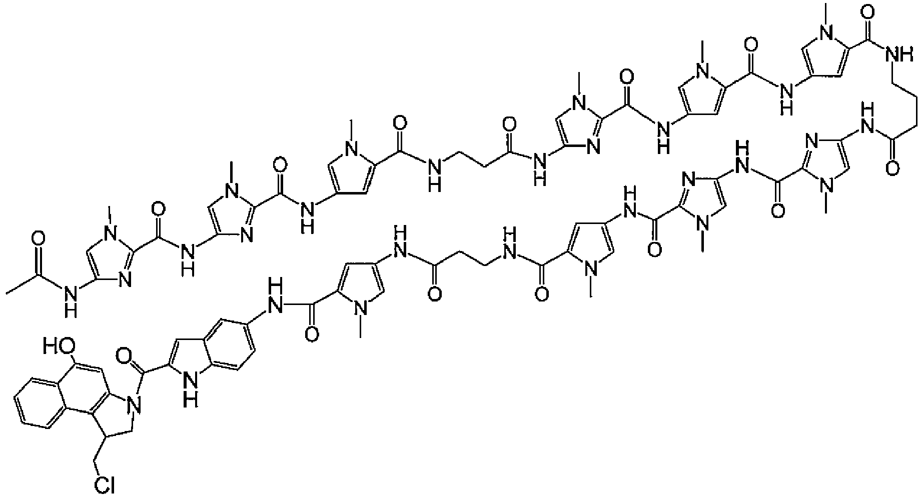

10. The conjugate according to claim 1, wherein the conjugate is represented by the following formula:

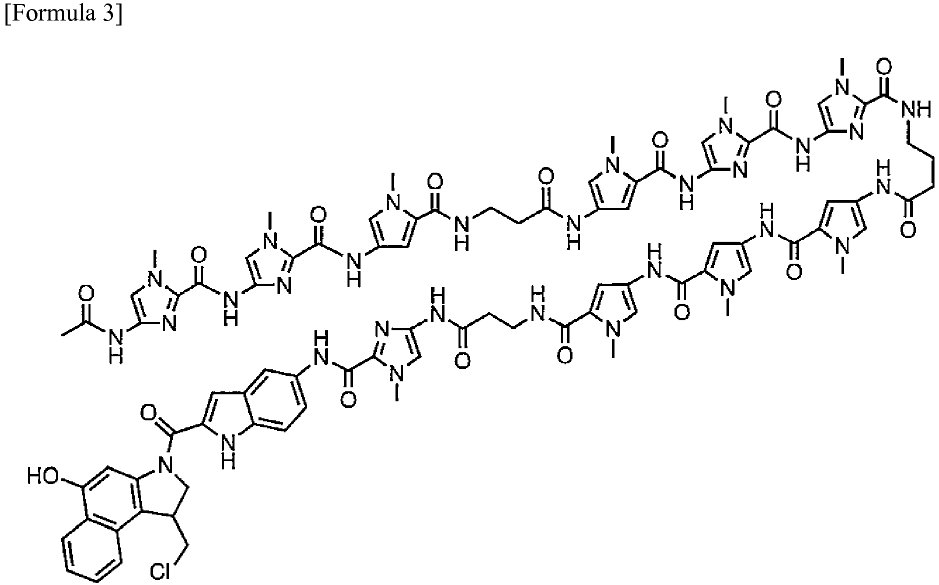

11. An alkylating agent that binds to a gene region encoding an immune checkpoint molecule, the alkylating agent comprising a conjugate according to any one of claims 8 to 10.

12. A pharmaceutical composition comprising a conjugate according to any one of claims 1 to 6 and 8 to 10.

13. The pharmaceutical composition according to claim 12, wherein the pharmaceutical composition is an anticancer agent.

14. A kit comprising a conjugate according to any one of claims 1 to 6 and 8 to 10.

15. A research reagent kit comprising a conjugate according to any one of claims 1 to 6 and 8 to 10.

16. A kit for treatment comprising a conjugate according to any one of claims 1 to 6 and 8 to 10.

17. A method for producing a conjugate that specifically alkylates a gene encoding an immune checkpoint molecule or a related molecule thereof, comprising the steps of:
(1) designing a DNA-binding compound so as to specifically bind to the gene encoding an immune checkpoint molecule or a related molecule thereof; and
(2) bonding the designed DNA-binding compound to an alkylating agent.

18. The method for producing a conjugate according to claim 17, wherein the immune checkpoint molecule is selected from the group consisting of PD-1, PD-L1, CTLA-4, CD80, CD86, PD-L2, and CD28, and the molecule related to the immune checkpoint molecule is at least one member selected from the group consisting of LAG3, TIGIT, IFNγ, IFN-I, IDO, KIR2DL-1, KIR2DL-2, KIR2DL-3, KIR2DS-1, KIR2DA-2, B7-H3 (CD276), TIM-3, VISTA, B7RP1, ICOS, B7-H4, HVEM, BTLA, CD137, CD70, OX40, CD40, CD137L, CD70L, OX40L, CD40L, GAL9, A2eR, TGFβ, IL1, IL6, IL10, IL12 and IL18, and immune-related molecules registered in gene databases.

19. The method for producing a conjugate according to claim 17 or 18, wherein the DNA-binding compound is selected from the group consisting of a bridged nucleic acid, a locked nucleic acid (LNA), PNA, pyrrole imidazole polyamide (PIP), a modified form of pyrrole imidazole polyamide (PIP), a DNA-binding protein, and a DNA-binding protein complex.

20. The method for producing a conjugate according to any one of claims 17 to 19, wherein the alkylating agent is a compound having a functional group having the ability to alkylate a particular nucleotide sequence of DNA.

21. The method for producing a conjugate according to claim 20, wherein the alkylating agent is secoCBI.
